# EUROPEAN PATENT SPECIFICATION

(11) **EP 0 833 912 B1**
(45) Date of publication and mention of the grant of the patent: **25.02.2009**
(21) Application number: 96918521.4
(22) Date of filing: 24.06.1996
(51) Int. Cl.: C07K 14/495, C07K 14/51, C07K 14/52, C07K 16/24, C12N 15/19

(54) **NOVEL TGF-BETA LIKE CYTOKINE**
NEUES TGF-BETA-AHNLICHES CYTOKIN
NOUVELLE CYTOKINE DE TYPE FACTEUR DE CROISSANCE TRANSFORMANT TGF-BETA

(30) Priority: 22.06.1995 AU PN370695; 23.08.1995 AU PN499095; 09.02.1996 AU PN798396
(43) Date of publication of application: 08.04.1998
(73) Proprietor: ST VINCENT'S HOSPITAL SYDNEY LIMITED, Darlinghurst, NSW 2010 (AU)
(72) Inventor: BREIT, Samuel, Norbert, Gordon, NSW 2072 (AU); BOOTCOV, Michelle, Bronte, NSW 2024 (AU)
(74) Representative: Harding, Charles Thomas
(86) International application number: PCT/AU1996/000386
(87) International publication number: WO 1997/000958

(56) References cited:
- EP-A- 0 904 292
- WO-A-94/03599
- WO-A-95/14772
- WO-A-96/18730
- AU-A- 1 830 195
- JP-A- 7 250 688
- JP-A- 7 258 293
- DATABASE EMBL [Online] 28 April 1995 (1995-04-28) HILLIER,L., CLARK,N., DUBUQUE,T., ELLISTON,K., HAWKINS,M.,: "The WashU-Merck EST Project" Database accession no. R33078 XP002199768
- GUIDEBOOK TO CYTOKINES AND THEIR RECEPTORS; Transforming Growth Factor-beta (TGFbeta), pp223-226.
- WAHL S.M.: 'TRANSFORMING GROWTH FACTOR BETA (TGF-BETA) IN INFLAMMATION: A CAUSE AND A CURE' JOURNAL OF CLINICAL IMMUNOLOGY vol. 12, no. 2, 01 March 1992, PLENUM PUBLISHING CO, US, pages 61 - 74, XP009001790

## Description

This invention relates to a novel TGF-β like cytokine and to isolated polynucleotide molecules encoding this protein. Particular applications of the invention may include treatments for wound and fracture healing, treatments and diagnostic assays for cancer, autoimmune and fibrotic diseases.

Macrophages play a central role in chronic inflammatory processes. The importance of these cells derives from the large variety of bioactive molecules that they produce and consequently, their capacity to amplify the inflammatory response. Their central role is also due to their capacity for communication with many other cells. For example, macropage derived platelet derived growth factor (PDGF) is an important growth factor for both fibroblasts and smooth muscle cells. Another group of proteins of great significance in the relationship of macrophages with various connective tissue cells (e.g. fibroblasts, smooth muscle, endothelium osteoblasts etc) are the TGF-β superfamily cytokines, especially the TGF-β proteins themselves.

The TGF-β superfamily consists of growth and differentiation factors that share substantial structural homology (1). In vertebrates, individual families comprise the TGF-β proteins themselves, the growth and differentiation factors (GDF)(embryonic growth and development), the bone morphogenetic proteins (BMP)(induce cartilage and bone formation), the inhibins and activins (regulate FSH secretion by pituitary), and mullerian inhibitory substance (MIS)(regression of Mullerian duct during male sex differentiation). These proteins share important structural features. Their bioactivity resides in the carboxyterminal region of 100-150 amino acids. Over this region, members of this superfamily share about 30% sequence identity to TGF-β1 and have 7 conserved cysteine residues. Within individual subgroups of the superfamily, proteins share 70% to 90% identity over the bioactive carboxy terminal domain. All superfamily members are thought to be cleaved at a cluster of basic residues 110 to 140 amino acids from the carboxy terminus of a precursor protein. Processing occurs immediately following a conserved RXXR sequence.

The three human TGF-β proteins share 80% sequence similarity over the bioactive portion of the molecule. The pro peptide (called latency-associated peptide (β1-LAP)) displays less than 50% similarity between family members. The β1-LAP is cleaved from the mature protein, but remains disulphide bonded to it. Separation of the β1-LAP is necessary to achieve biological activity (2).

The TGF-β proteins have been studied intensively because of their biological importance and therapeutic potential. Their biology and functions are well known and have been extensively reviewed (e.g. 2, 3, 4). In general terms they promote differentiation and differentiated function in a wide variety of cells. They are potent chemotactic factors for macrophages and fibroblasts and generally inhibit cell proliferation, perhaps because of their role in differentiation. In the context of inflammation, TGF-β is a potent stimulator of fibroblast collagen and matrix protein synthesis, promotes angiogenesis, modulates expression of adhesion molecules and inhibits lymphocyte proliferation, production of some lymphokines and NK cell function. This molecule has been of great interest to the pharmaceutical industry mainly, because of its demonstrable capacity to promote wound and fracture healing in vivo. TGF-β has also been heavily implicated in the pathogenesis of chronic inflammatory processes and mechanisms. Wahl. SM. teaches transforming growth factor beta in inflammation (3). Further, its local production has been used as a surrogate marker e.g. in active fibrotic diseases such as cirrhosis and it therefore has potential in the diagnostic arena.

The present inventors have now isolated a polynucleotide molecule including a novel cytokine gene, clone 13 (CL13), which encodes a dimeric protein (pCL13) that appears to represent the first member of a new class of protein within the TGF-β superfamily.

The claims describe the specific embodiments of the present invention.

Herein described is an isolated polynucleotide molecule comprising a nucleotide sequence encoding, or complementary to a sequence encoding, a protein designated pCL 13 or a biologically active fragment thereof.

The isolated polynucleotide molecule may comprise a nucleotide sequence the same as that of the CL13 clone described herein or may contain single or multiple nucleotide substitutions and/or deletions and/or additions thereto. The nucleotide substitutions which are envisaged may result in one or more conservative or non-conservative amino acid substitution(s). By conservative substitutions, the intended combinations are - G,A; V,I,L,M; D,E; N,Q; S,T; K,R,H; F,Y,W,H; and P,Nα-alkylamino acids. The term "nucleotide sequence" also includes sequences with sufficient homology to hybridise with the nucleotide sequence under medium or, more preferably, high stringency conditions (5) and to nucleotide sequences encoding functionally equivalent sequences. In addition, the term "nucleotide sequence" includes sequences having at least 70%, more preferably 90%, homology to clone 13 described herein or any portion thereof of > 10 nucleotides in length.

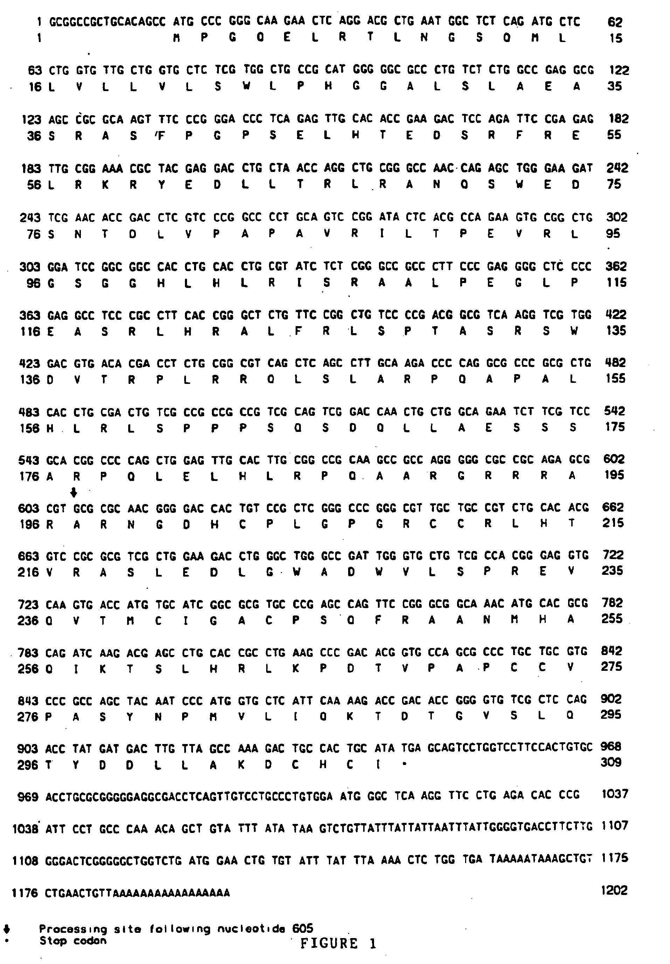

Most preferably, the isolated polynucleotide molecule comprises a nucleotide sequence substantially corresponding to the nucleotide sequence shown in Figure 1 or a portion thereof, or a complementary sequence thereto. The term "portion thereof, in this regard, is to be understood as referring to portions of the nucleotide sequence which encode biologically active protein fragments and also, to portions of the nucleotide sequence, preferably > 10 nucleotides in length, which may be used in, or for the production of probes useful for, hybridisation assays.

Also described herein are oligonucleotide primers for the above sequences, antisense sequences and homologues of said primers and antisense sequences, complimentary ribozyme sequences, catalytic antibody binding sites and dominant negative mutants of the polynucleotide molecule.

Herein described is a protein designated pCL13, or a biologically active fragment thereof, in substantially pure form.

Preferably, the protein, or biologically active fragment thereof, comprises a monomeric polypeptide having an amino acid sequence substantially corresponding to the amino acid sequence shown in Figure 1 or a fragment thereof.

Biologically active fragments thereof as described herein refers to monomeric pCL13 polypeptides (with or without the propeptide) and other polypeptide or peptide portions (whether monomeric or dimeric) thereof which may consist of sequences which inhibit, mimic or enhance the biological effect of the protein and dominant negative protein mutants, binding proteins including soluble receptors, other protein and/or glycosaminoglycans. The pCL 13 propeptide may also represent a biologically active fragment of pCL13.

The protein, or biologically active fragment thereof, as described herein may be purified from natural sources (e.g. lungs, skin etc) or cell lines, or may be produced recombinantly by any of the methods common in the art (5).

Herein described is an non-human organism transformed with the polynucleotide molecule described herein.

The organisms which may be usefully transformed with the polynucleotide molecule described herein include bacteria such as *E.coli* and *B.subtilis,* eukaryotic cell lines such as CHO, fungi, yeast, non-human animals and plants.

Transformed or transgenic, non-human animals may be established to, for example, overexpress CL13, pCL13 or a biologically active fragment thereof or, alternatively, generate antisense or ribozyme RNA molecules to inhibit native CL13 expression.

Herein described is an antibody or fragment thereof specific to pCL13 or an antigenic portion thereof. The antibody may be polyclonal or monoclonal and may be produced by any of the methods common in the art.

It is also to be understood that the invention relates to kits for diagnostic assays, said kits comprising an antibody as described herein or nucleotide primers for PCR based assays.

Herein described is a protein or antigenic portion thereof, capable of binding to an anti-pCL13 antibody.

pCL13 is suitable for *in vivo* and *in vitro* procedures involving both human and animal cells. pCL13 is also suitable for both medical and veterinary use. In particular, pCL 13 may be suitable for methods of treatment for any disease or condition beneficially treatable with TGF-β or another member of the TGF-β superfamily.

Herein described is a method of treatment to assist wound and/or fracture healing and/or ischaemic injury, comprising administering (for example, orally, topically, intravenously or subcutaneously) to a subject a preparation comprising a protein, or biologically active fragment thereof, as described herein, optionally in admixture with a suitable pharmaceutically acceptable carrier.

The protein, or biologically active fragment thereof, as described herein, may also be useful for one or more of the following:
(i) Immunosuppression and anti-inflammatory effects for conditions such as autoimmune diseases or transplantation;
(ii) Down regulation of leukocyte extravasation and motility in infective or inflammatory processes; and
(iii) Treatment of tumours through promotion of differentiation and antiproliferation action.

Such uses may be achieved by administration of the protein, or a biologically active fragment thereof, to a subject, or by gene therapy using all or part of the polynucleotide molecule described herein. Such gene therapy may be used to, for example, establish overexpression of CL13, or pCL13 or a biologically active fragment thereof in the host cell or, alternatively, to generate antisense or ribozyme RNA molecules to inhibit native CL13 expression.

It is also possible that inhibiting the action of pCL13 may provide treatment of fibrotic/fibroproliferative disorders such as rheumatoid arthritis, atherosclerosis, pulmonary fibrosis, scleroderma, liver cirrhosis and keloids, and inhibition of tumour immunosuppression associated with conditions such as tumours, infections (especially viral) and chronic inflammatory diseases. These treatments may be achieved by using:
fragments or peptides of the pCL13 protein that inhibit receptor binding;
binding proteins for pCL13 including soluble receptors for this molecule, glycosaminoglycans, and other molecules which may inhibit or destabilise receptor ligand interaction;
antibodies directed at pCL13 or its receptor;
antisense or ribozyme strategies in which expression or stability of the pCL13 gene product is disturbed;
dominant negative mutants of the CL13 gene which, when expressed in a host cell, will destabilise or affect the activity of pCL13. (As the pCL13 protein is a dimer, a second gene product which has been modified may bind to the native pCL13 to form a heterodimer). Thus, an appropriately modified pCL13 variant may essentially render the pCL13 inactive through mechanisms such as enhanced degradation, aberrant intracellular trafficking and inhibition of export from the cell and inhibition of bioactivity.

Thus, herein described is a heterodimeric protein comprising a monomeric polypeptide of pCL13 together with a monomeric polypeptide of another protein from the TGF-β superfamily.

pCL13 or biologically active fragments thereof may be formulated into standard pharmaceutical compositions suitable for the administration of proteins. Suitable formulations can be found, for example, in Remington's Pharmaceutical Sciences, latest edition, Mack Publishing Company, Easton, PA.

The dosage levels of pCL13 or biologically active fragments thereof may be comparable to those useful for other members of the TGF-β superfamily. These levels are well understood in the art, and the precise dosage can be adjusted according to the condition of the subject, the mode of administration, and the judgement of the attending physician.

Possible diagnostic applications include diagnosis of cancer, inflammatory and fibrotic disorders such as rheumatoid arthritis, cirrhosis and altherosclerosis in which enhanced synthesis of this gene may be present.

To facilitate the abovementioned applications for pCL13, it will be necessary to produce the protein in large quantities. However, extensive studies with other protein members of the TGF-β superfamily, has revealed a number of difficulties in achieving expression in commercial amounts. For instance, expression in simple prokaryotic systems are largely unsuitable since members of the superfamily are cysteine knot dimeric proteins having a complex pattern of disulphide bond linkages.

The current strategy for expression of TGF-β superfamily proteins is therefore to express the whole protein from a suitable DNA construct transfected into mammalian cells. However, this strategy necessitates treatment of the culture supernatant to separate processed (cleaved) bioactive mature protein from the propeptide and unprocessed (uncleaved) material. This creates additional costs and difficulties because some of the expressed material is non-productive as typically 30%-50% of the secreted material will not be appropriately cleaved. The additional chromatographic procedure also generates extra losses of protein and incurs additional cost and time.

Previous efforts to express the mature bioactive portion of these proteins alone, has been unsuccessful, indicating that the propeptide is essential for achieving expression and secretion.

The present inventors, however, have been unexpectedly able to achieve expression and secretion of pCL13 without expressing the leader or propeptide, using transfected mammalian cell cultures.

Thus, herein described is a method for producing a protein designated pCL13 or a biologically active fragment thereof, comprising transforming a suitable non-human host organism with a polynucleotide molecule comprising a nucleotide sequence encoding pCL13 or a biologically active fragment thereof, wherein said polynucleotide molecule is constitutively or inducibly expressed in said host organism.

Preferably, the nucleotide sequence encoding the pCL13 or a biologically active fragment thereof, does not comprise sequence encoding the leader or propeptide of pCL 13.

In place of sequences encoding the native leader or propeptide, it may be preferable to include within the polynucleotide molecule sequences encoding a heterologous leader (e.g. the follicle stimulating hormone (FSH) leader sequence) to assist expression.

Suitable non-human host organisms transformed with the polynucleotide molecule described herein may be any of those mentioned above. However, preferred organisms include mammalian cell lines, yeast (e.g. Pichia and Saccharomyces) and non-human animals.

Expression of only the mature bioactive portion of pCL13 thereby provides the following advantages:
(i) Higher levels of expression; and
(ii) No necessity to purify from propeptide and unprocessed full length CL13 protein.

Further, since it is not necessary to express the whole protein, it is possible and simple to add amino-terminal epitope tags (e.g. FLAG and/or HIS) that can significantly assist with the purification and visualisation of recombinant protein.

Also, the capacity to express the mature bioactive portion of pCL13 in mammalian cells, indicates that it will also be able to be readily expressed in yeast strains, such as the *Pichia pastoris* which is capable of secreting disulphide linked proteins. Production of protein in yeast is much cheaper and easier than production by mammalian cells.

The invention will now be further described by way of the following non-limiting examples and with reference to the accompanying figures.

### Brief Description of the Figures

Figure 1 provides the nucleotide sequence and putative amino acid sequence of clone 13 encoding pCL13.
Figure 2 shows CL13 expression in macrophage cultures.
   Panel A. 15 µg total RNA was loaded per lane. Macrophage treatments were: lane 1, no treatment; lane 2, 1,000 U IFNγ overnight, lane 3, 1 µM retinoic acid overnight; lane 4,1 µM retinoic acid overnight followed by 10 µg/mL LPS for 3 hours; lane 5, 10 µg/mL LPS for 3 hours.
   Panel B. 20 µg total RNA was loaded per lane. Macrophage treatment were: lane 1, 1 µM retinoic acid for 3 days followed by 10 µg/ml LPS for 3 hours; lane 2,1 µM retinoic acid overnight followed by 50 nM PMA for 3 hours; lane 3, 50 nM PMA for 3 hours; lane 4, untreated macrophages.

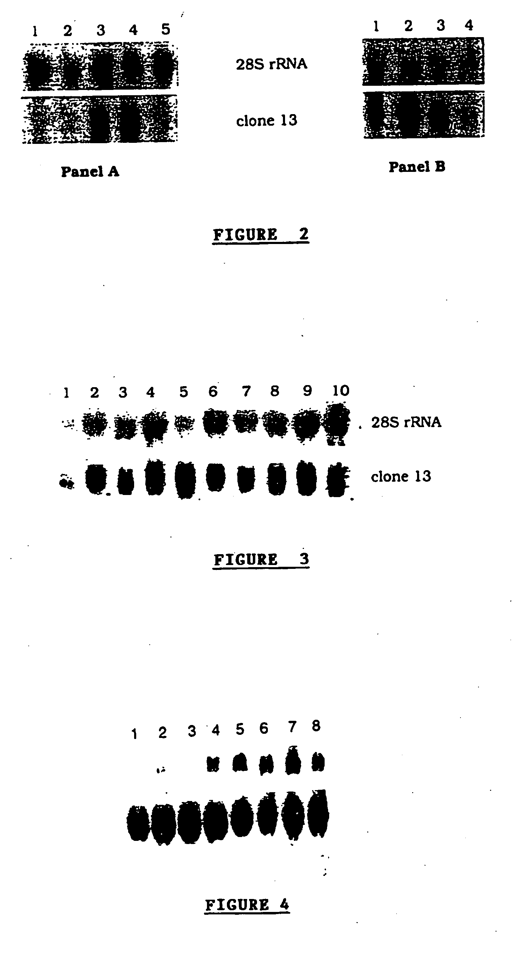

Figure 3 shows a northern blot analysis of clone 13 expression from macrophages treated with cytokines. All treatments were 3 hours. Lane 1, untreated macrophages; lane 2, 50 nM PMA; lane 3, 50 U/mL GM-CSF; lane 4, 100 U/mL M-CSF; lane 5, 100 U/mL IL1-β; lane 6, 10ng/mL TGF-β; lane 7, 10 U/mL PDGF-BB; lane 8, 50 U/mL IL-2; lane 9, 100 U/mL TNF-α, lane 10, 50 U/ml IL-6.
Figure 4 shows a northern blot analysis of the expression of clone 13 in U937. 20 µg total RNA was loaded per lane on a 1.2% agarose denaturing formaldehyde gel. Lane 1, no treatment; lane 2, 1 µM retinoic acid for 3 days, lanes 3, 4, 5, 6, 7, 1 µM retinoic acid for 3 days followed by 160 nM PMA for 20 min, 1 h, 2 h, 3 h and 12 h respectively; lane 8, 160 nM PMA for 3 h. Probes were labelled with ³²P. The blot was hybridized at 65°C and subjected to post hybridization washes and autoradiography.

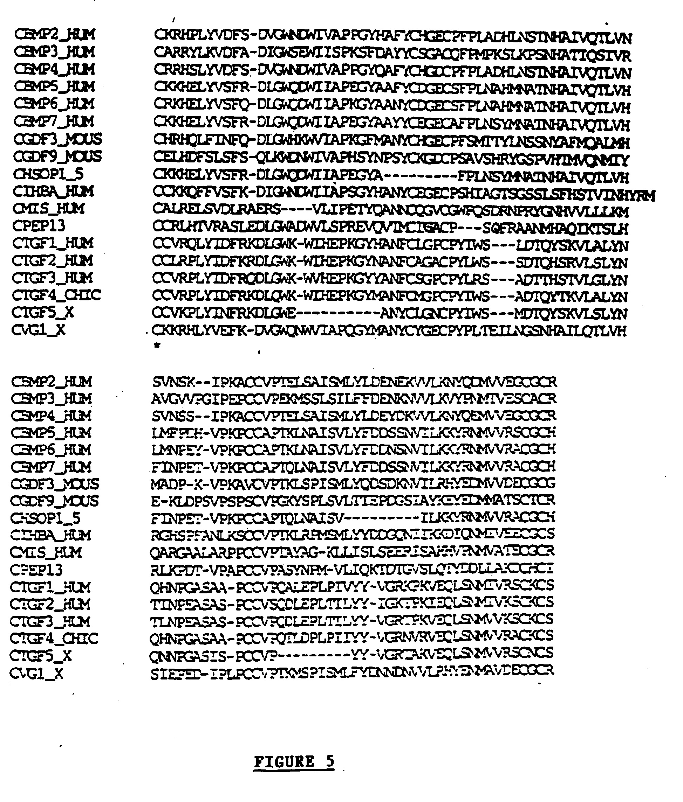

Figure 5 provides a multiple sequence alignment of the carboxy terminal halves of pCL13 and other TGF-β superfamily members.

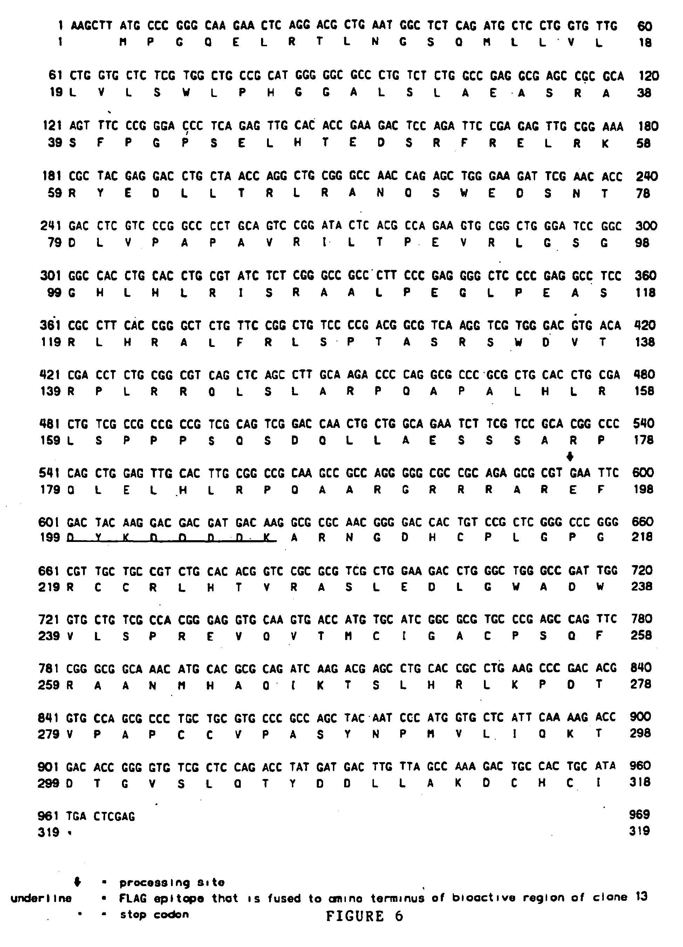

Figure 6 provides the nucleotide sequence and putative amino acid sequence for clone 13 in construct C13LB. The coding region for the bioactive portion of pCL13 commences with nucleotide 625.

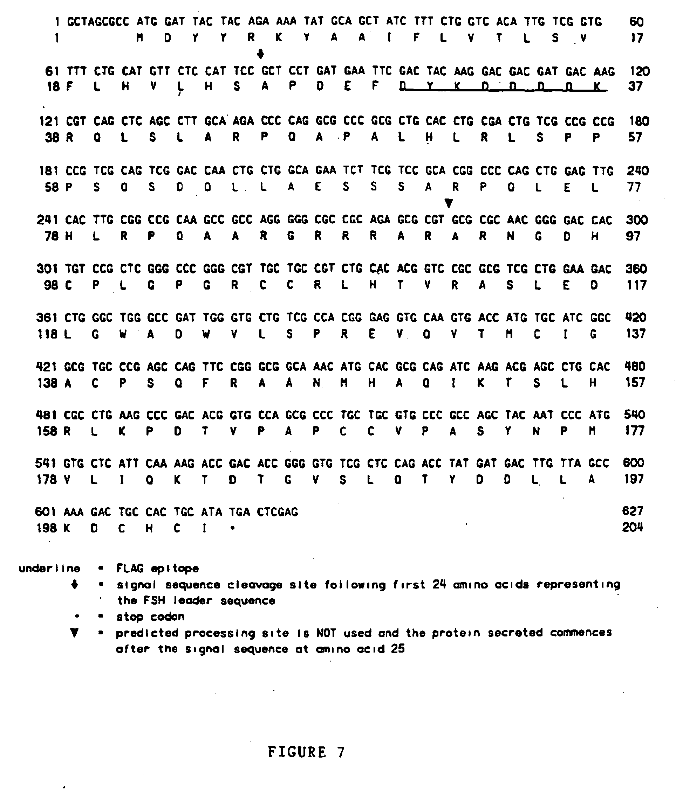

Figure 7 provides the nucleotide sequence and putative amino acid sequence for clone 13 in construct FFC13S. The predicted bioactive portion of pCL13 commences with amino acid 92.

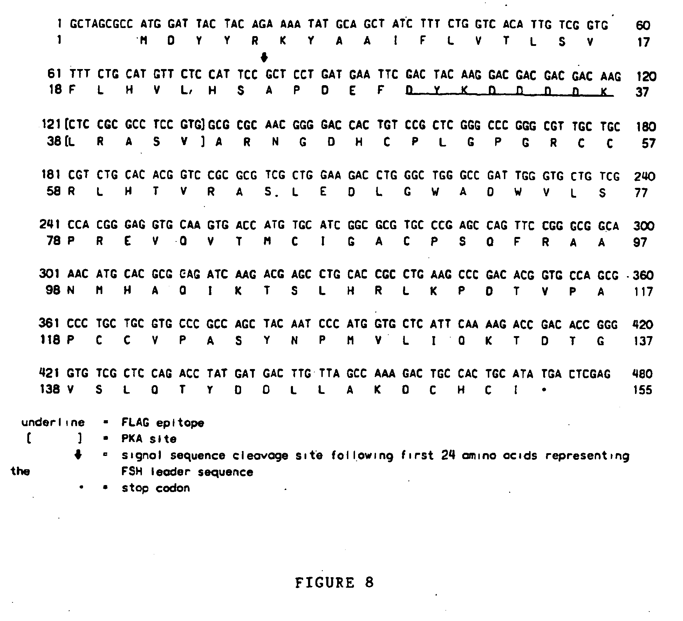

Figure 8 provides the nucleotide sequences and putative amino acid sequence for clone 13 in construct C13SA. The coding region for the bioactive portion of pCL13 commences with nucleotide 136.

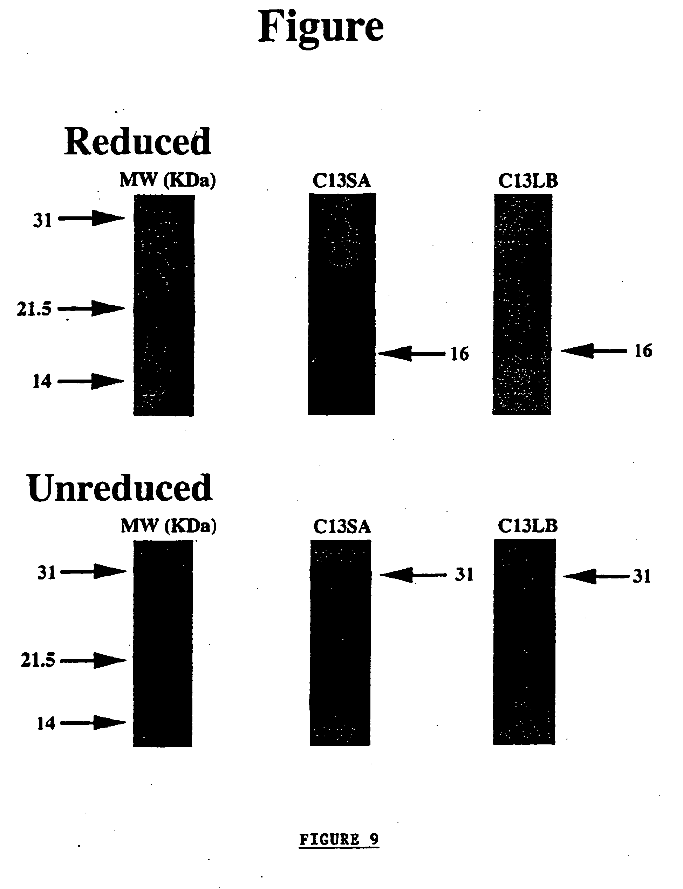

Figure 9 shows a Western blot of purified recombinant pCL13 (FFC13S construct) visualised with anti-FLAG antibody.

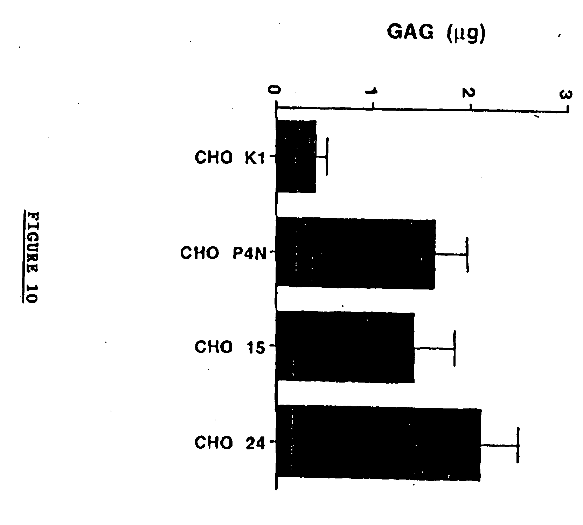

Figure 10 provides a graph of the results obtained from glycosaminoglycan analysis in non-transfected (K1) and CL13 (FFC13S construct) transfected (P4N, 15 and 24) CHO cells using dimethyl-methylene blue (DMB) assay.

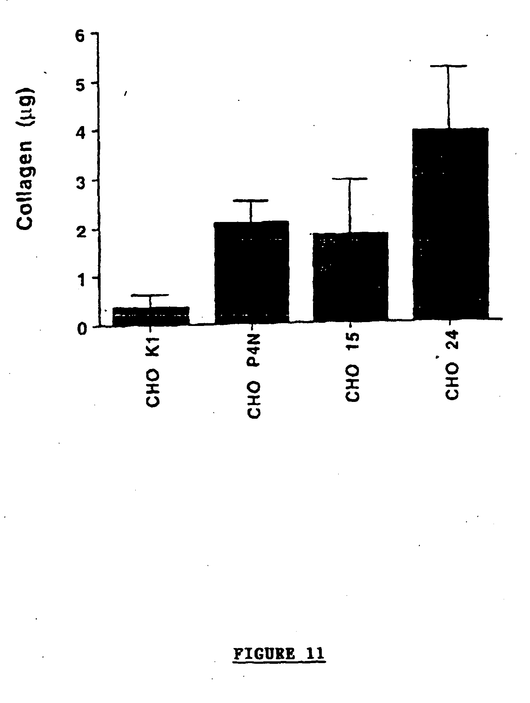

Figure 11 provides a graph of results obtained from collagen production assays of non-transfected (K1) and CL13 (FFC13S construct) transfected (P4N, 15 and 24) CHO cells.

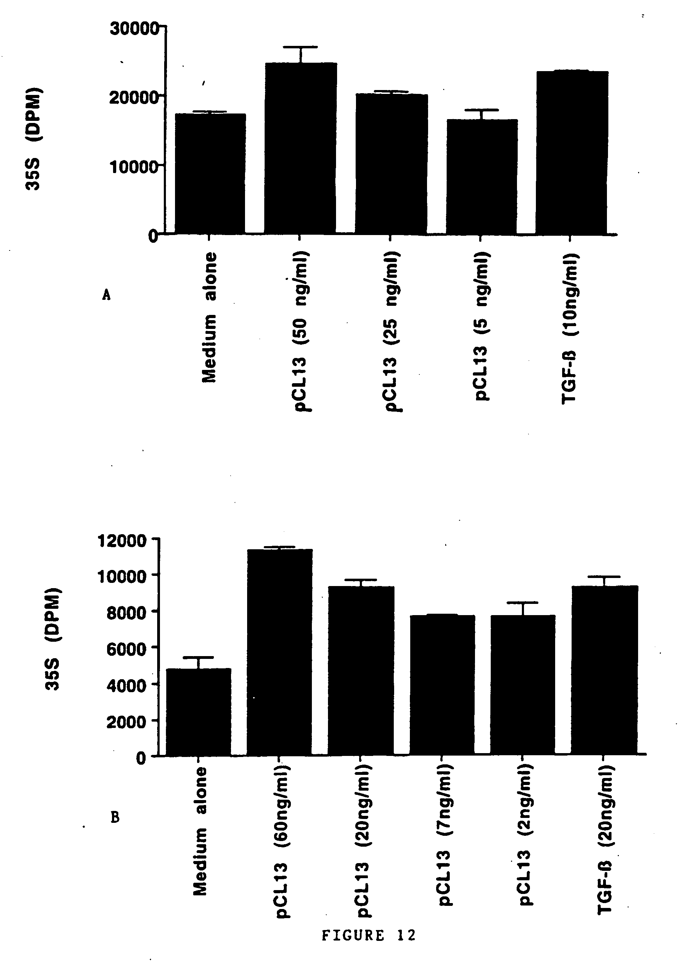

Figure 12 provides graphs of results obtained from glycosaminoglycan production analysis in 3T3 (Figure 12A) and CCD (Figure 12B) cells following addition of various concentrations of pCL13 (expressed from construct C13SA).

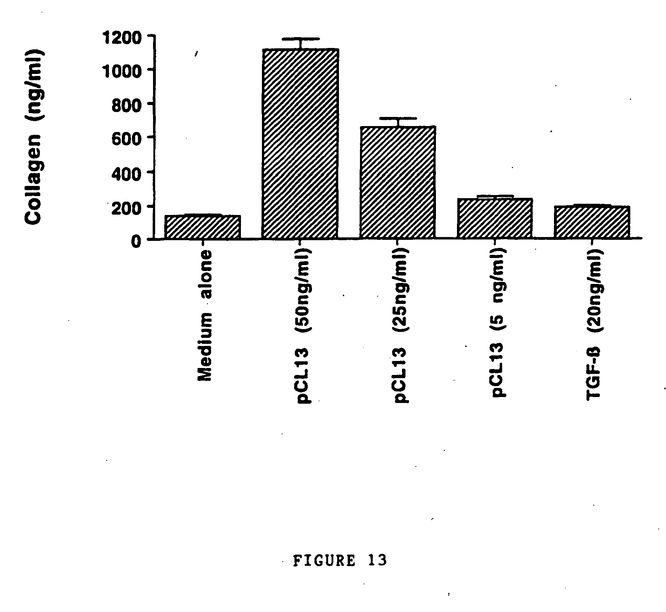

Figure 13 provides graphical results obtained from collagen production analysis in CCD cells following addition of various concentrations of pCL13 (expressed from construct C13SA).

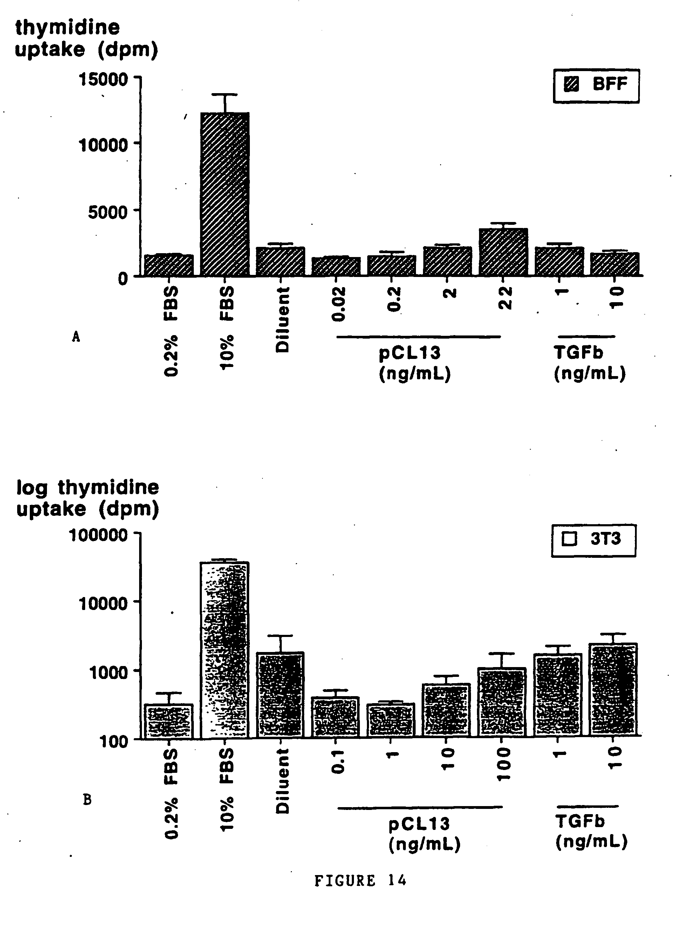

Figure 14 provides graphs of results showing growth factor activity under limiting serum conditions of pCL13 (expressed from construct C13SA) against TGFβ in human baby foreskin fibroblasts (BFF) (Figure 14A) and 3T3 cells (Figure 14B).

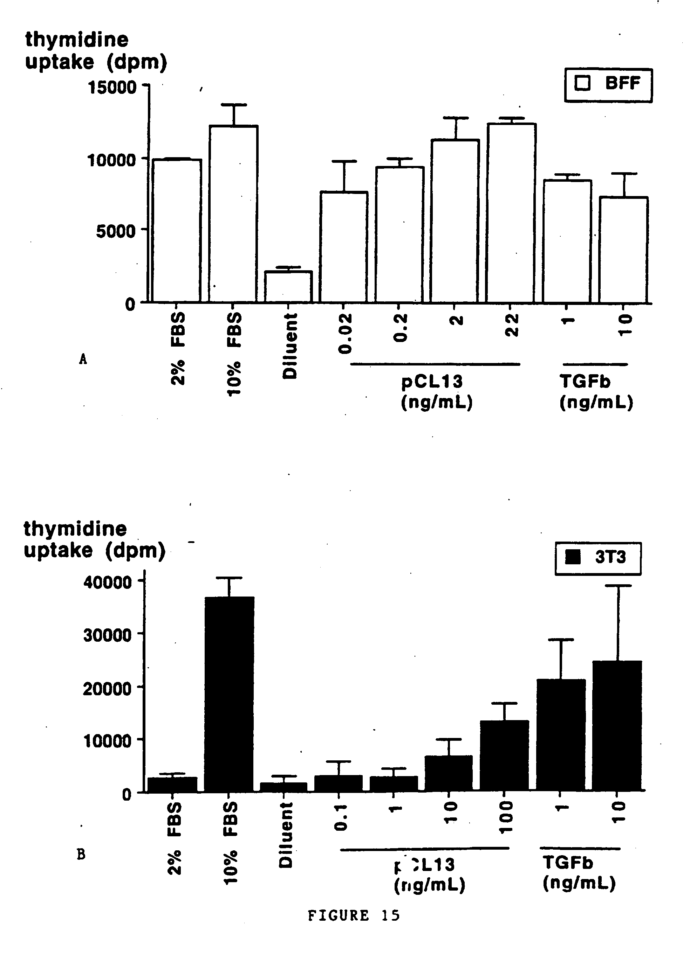

Figure 15 provides graphs of results showing growth factor activity in the presence of serum of pCL13 (expressed from construct C13SA) and TGFb in BFF and 3T3 cells.

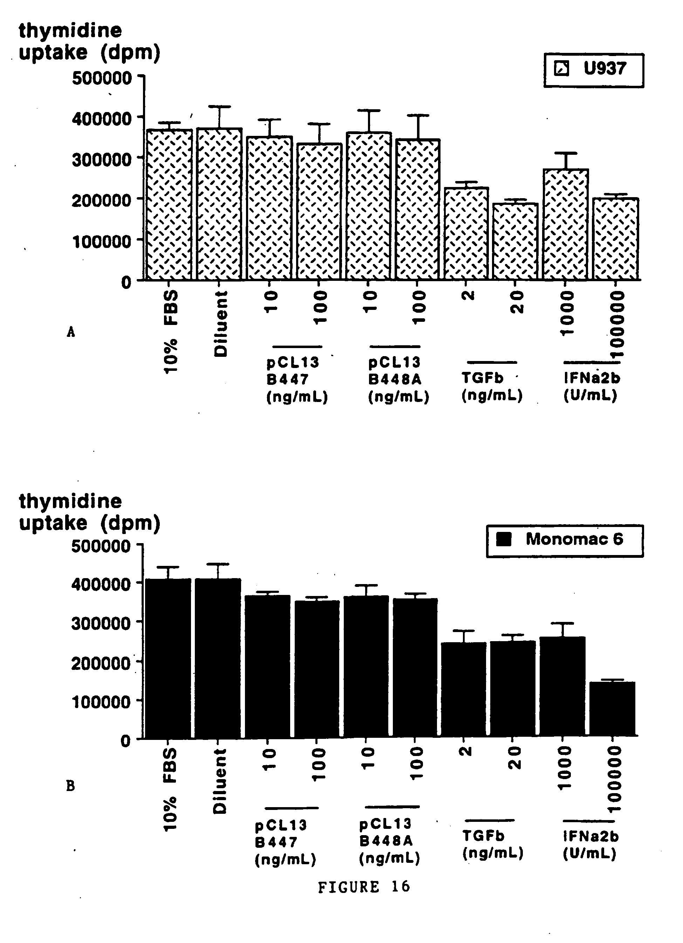

Figure 16 provides graphs of results showing the effect of pCL13 (expressed from construct C13SA) of pCL13. TGFβ and IFNa2b on the proliferation of U937 human monocytic cells (Figure 16A) and mono Mac 6 human monocytic cells (Figure 16B).

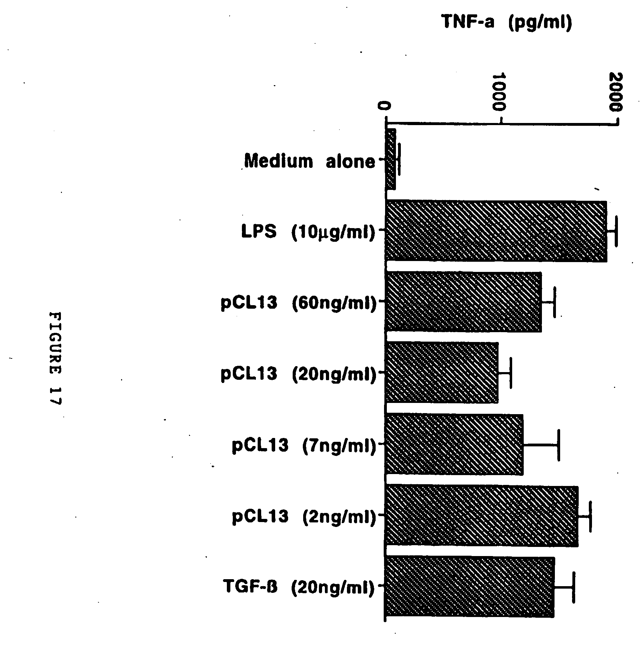

Figure 17 provides graphical results of an analysis of differing pCL13 (expressed from construct C13SA) concentrations on TNF-α production in human culture derived macrophages.

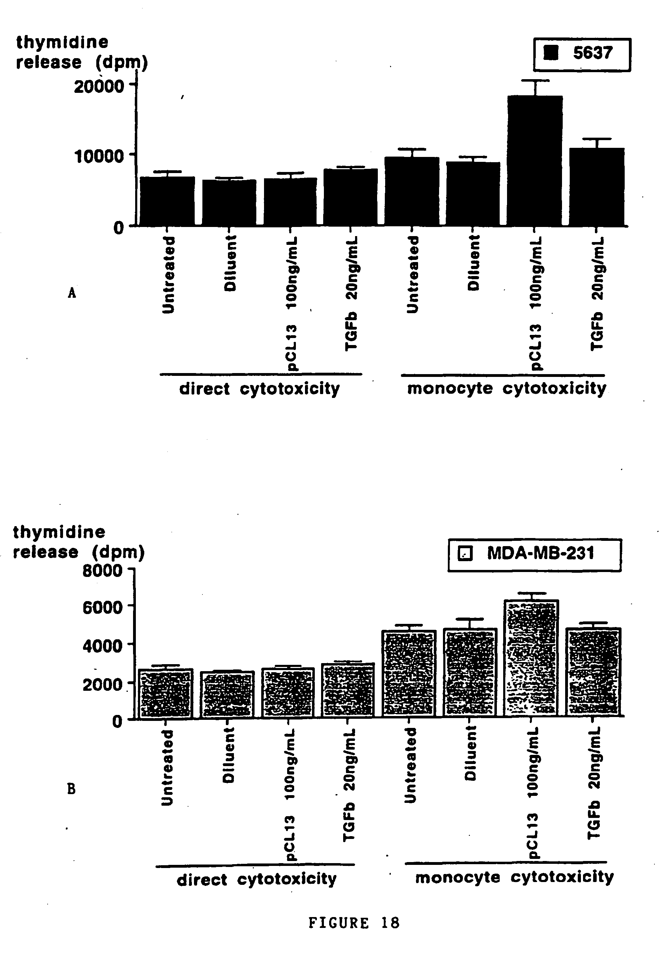

Figure 18 provides graphs of results showing the effect of pCL13 (expressed from construct C13SA) concentrations on the cytoxicity of monocytes towards 5637 bladder tumour target cells (Figure 18A) and MDA-MB-231 breast tumour target cells (Figure 18B).

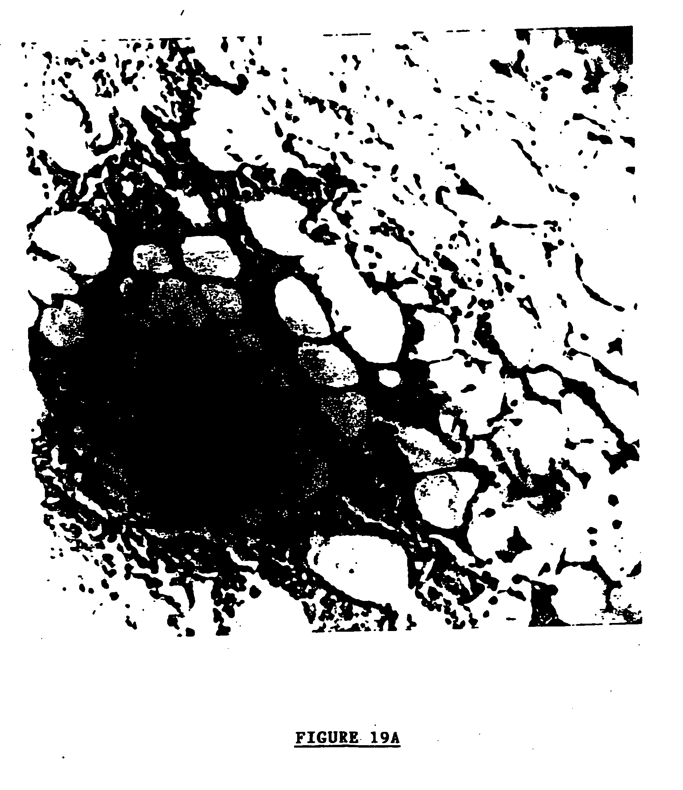

Figure 19A provides a micrograph of subcutaneous tissue taken from a rat having been administered pCL13 (expressed from construct C13SA).

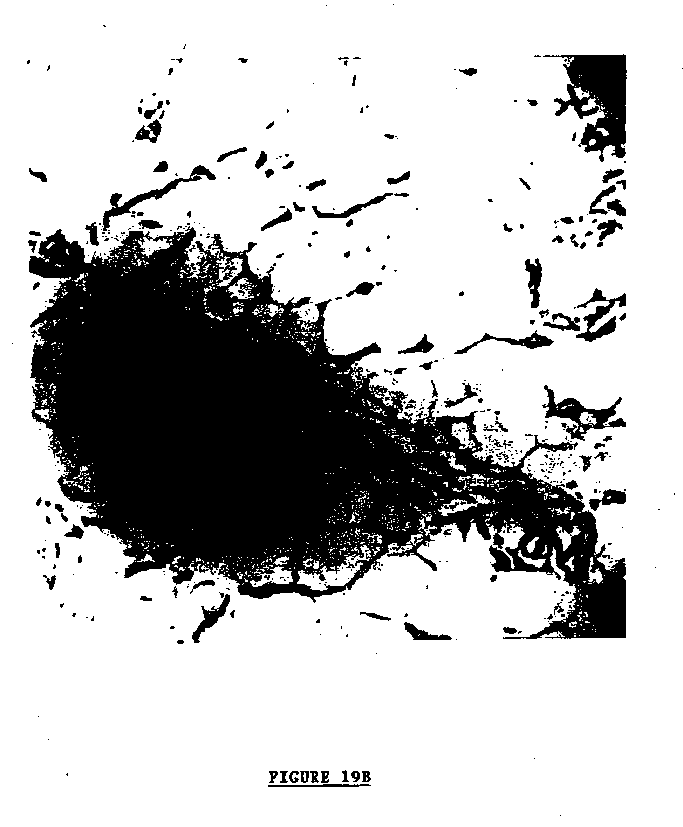

Figure 19B provides a micrograph of subcutaneous tissue taken from a control rat having been administered saline only.

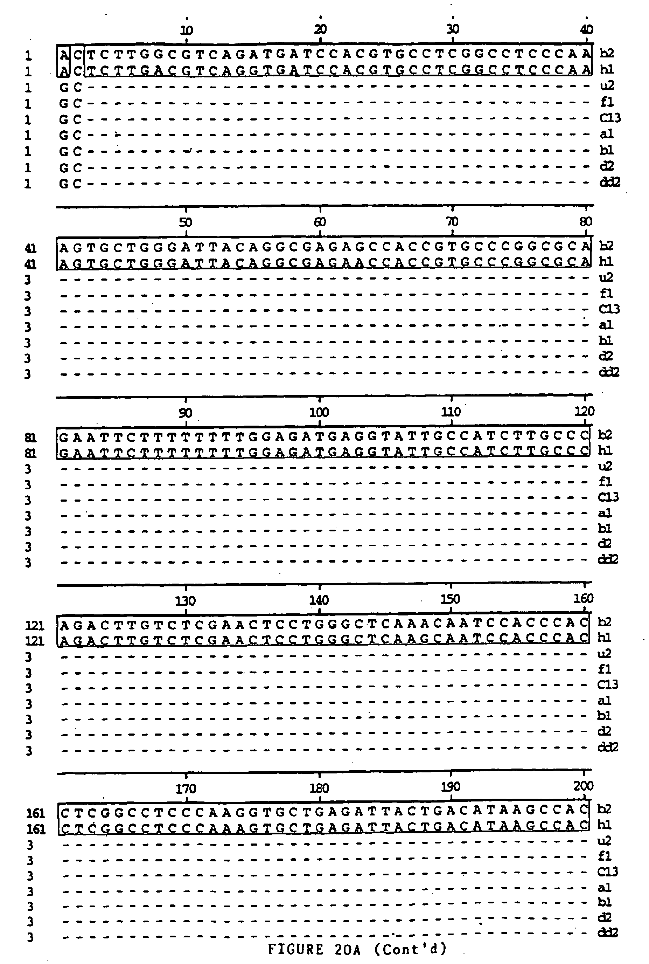

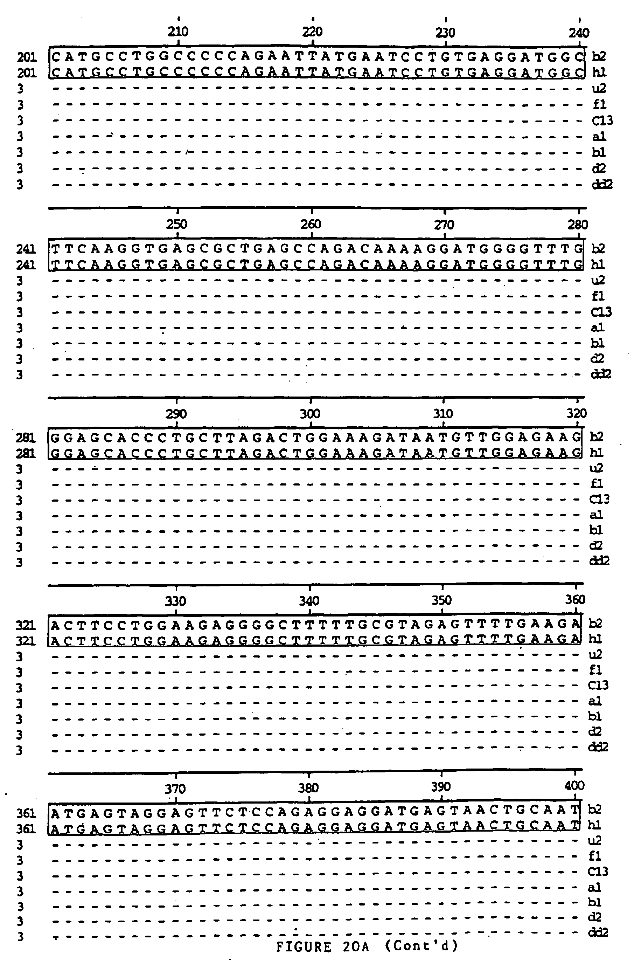

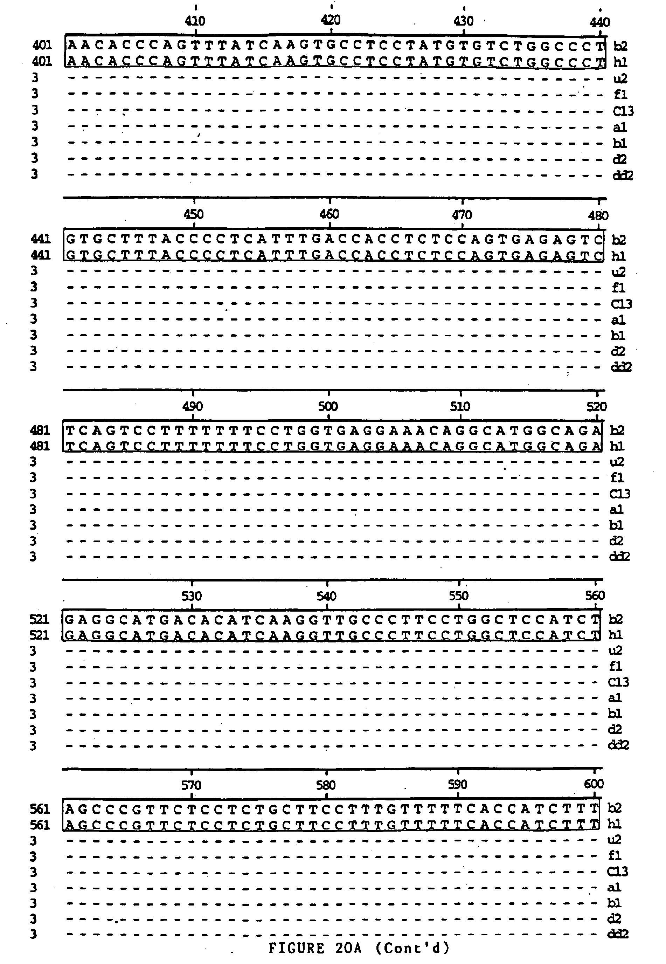

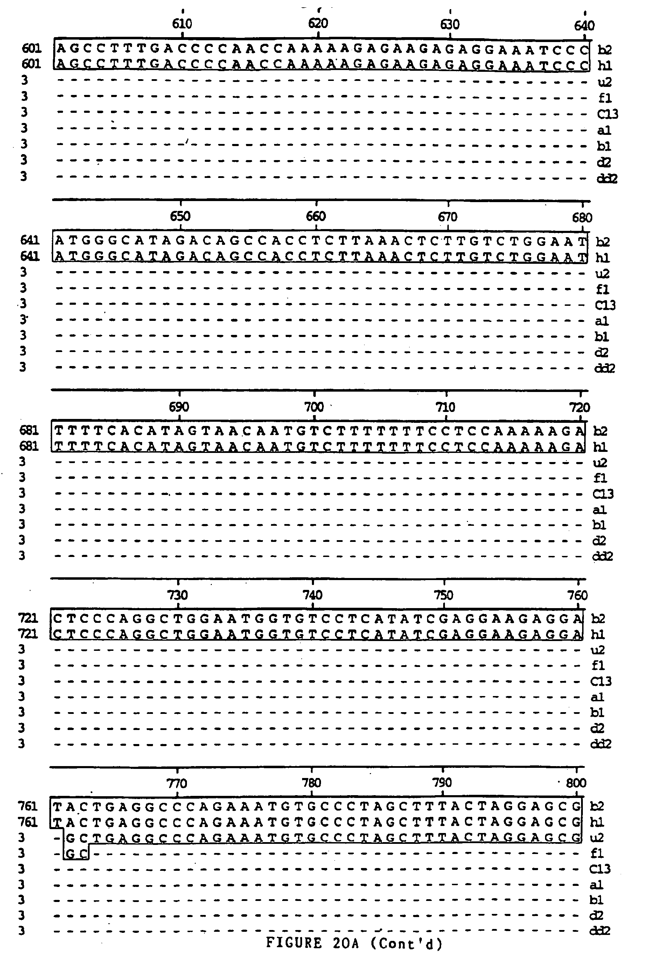

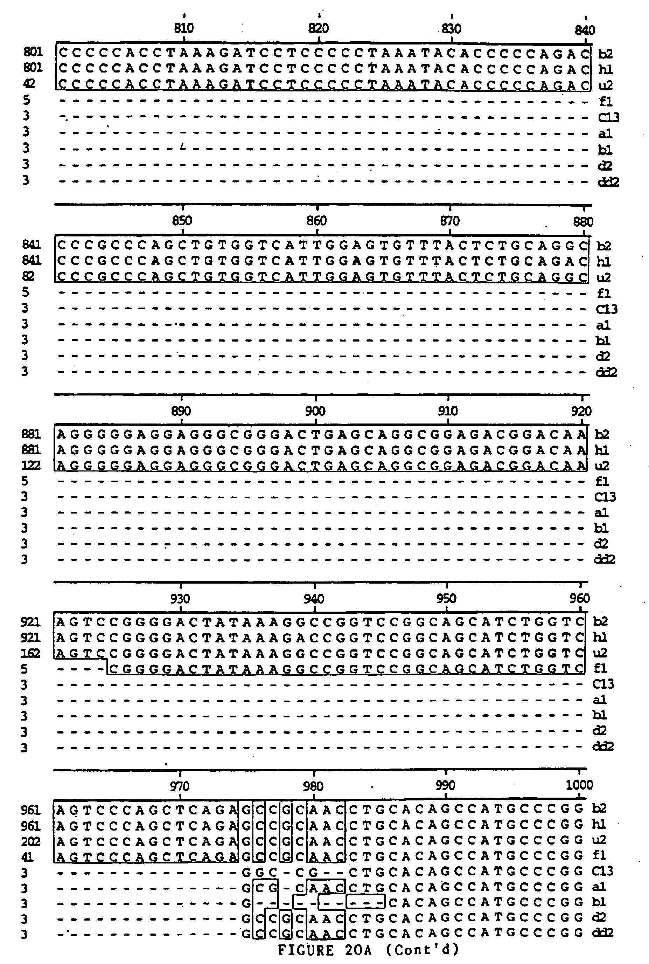

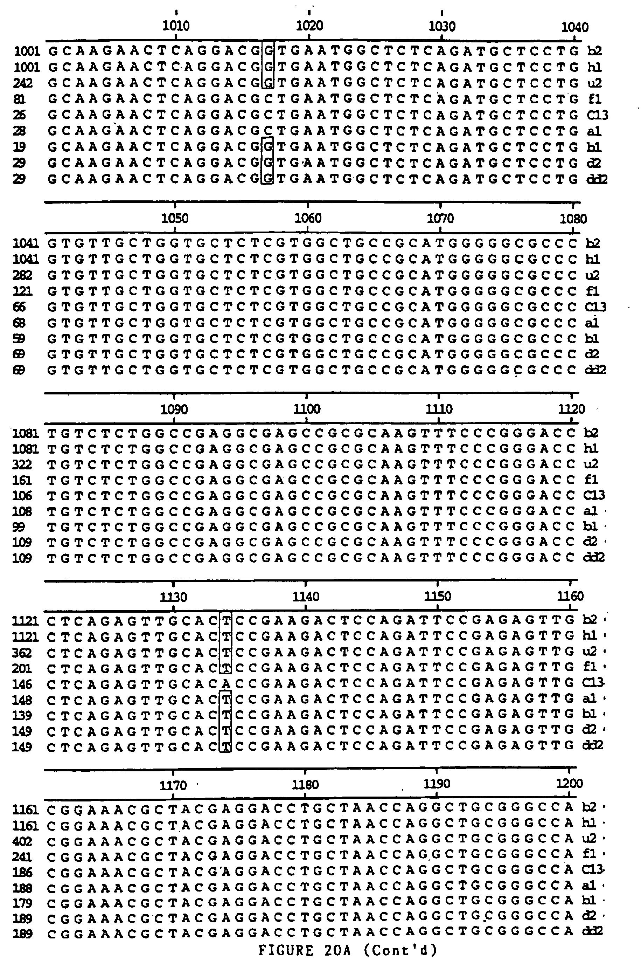

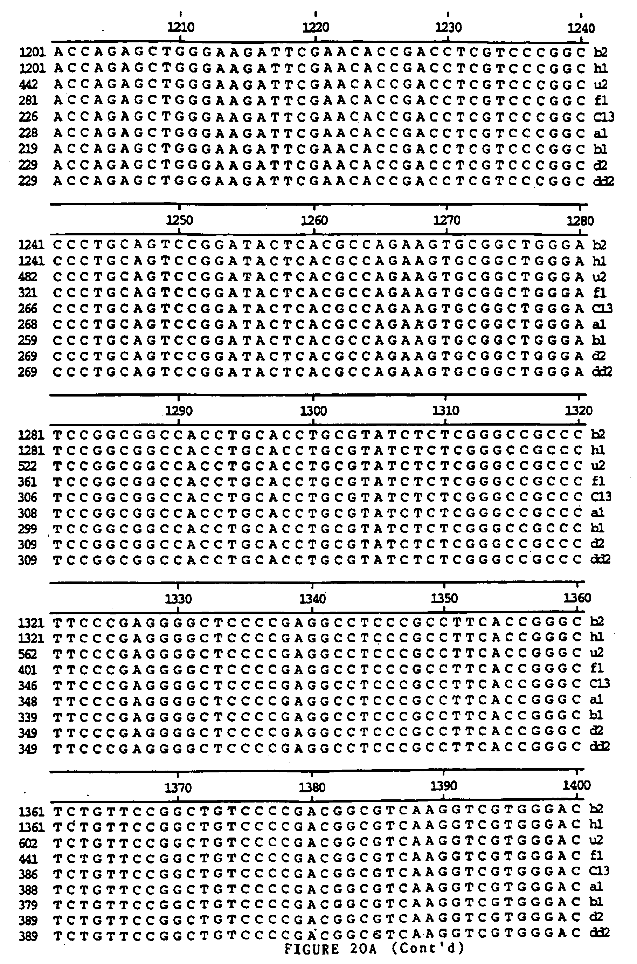

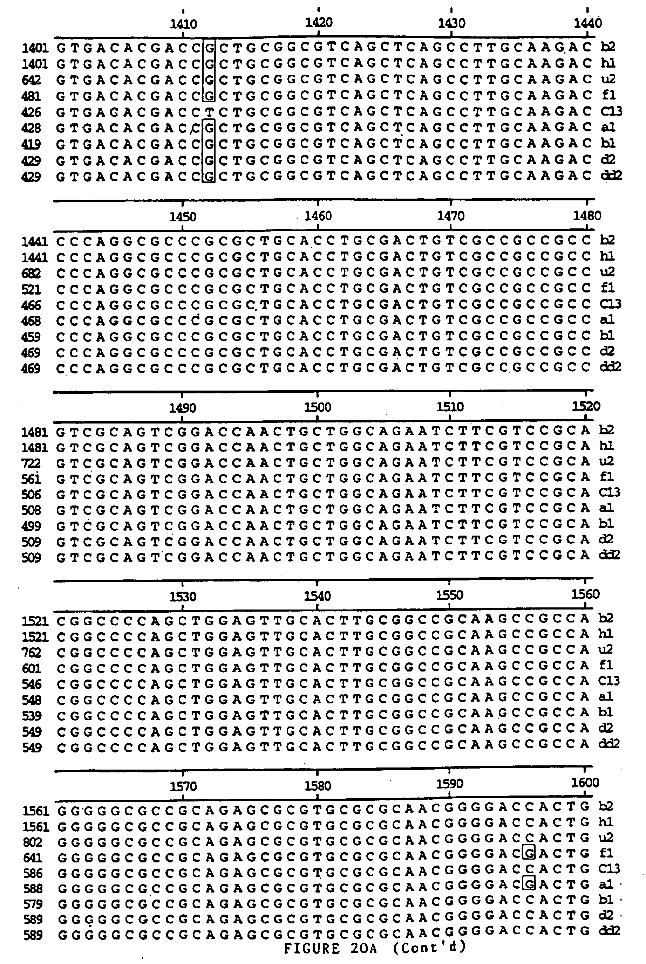

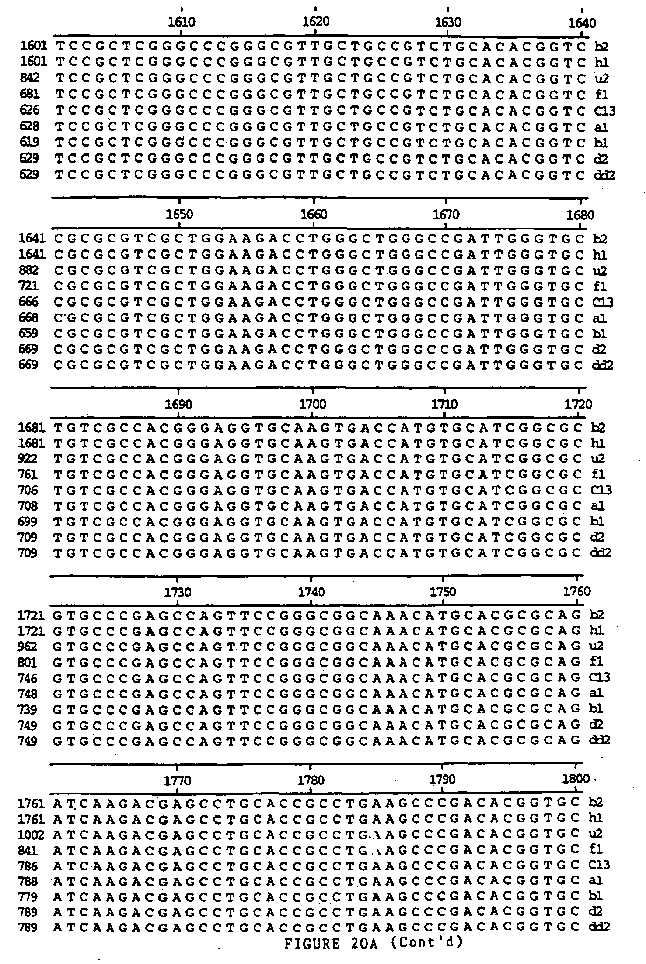

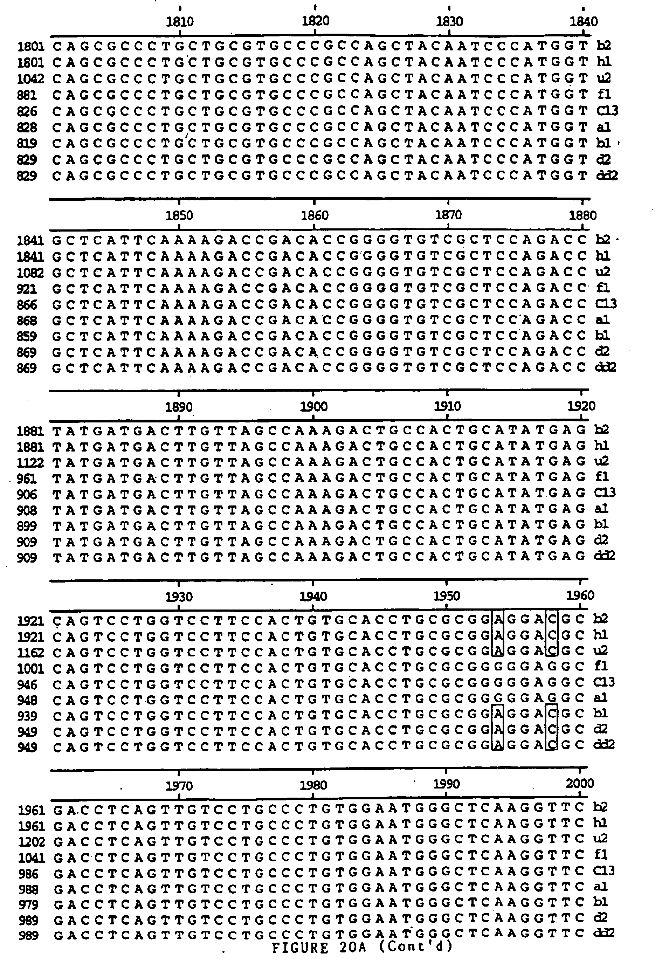

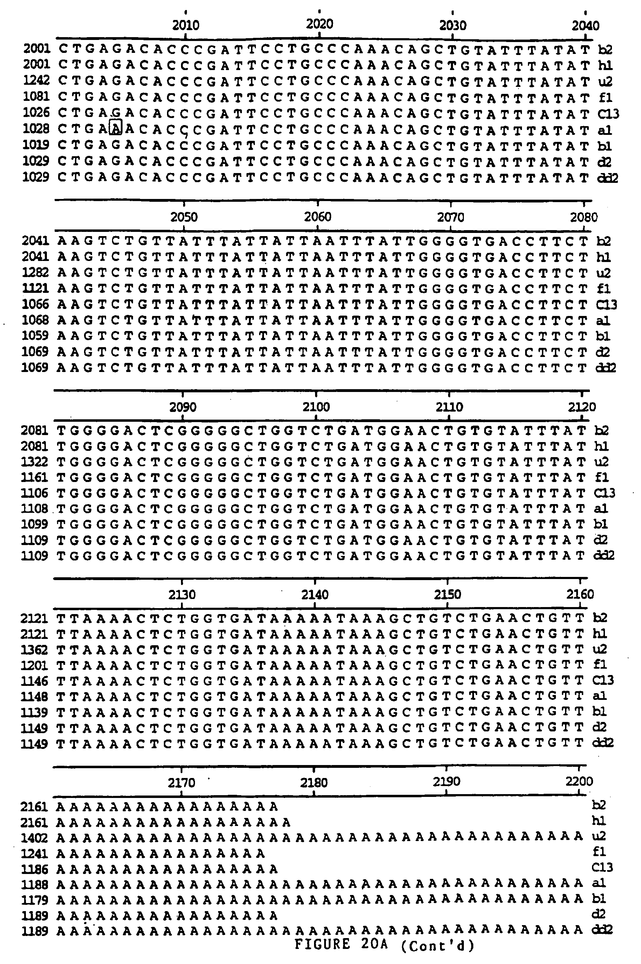

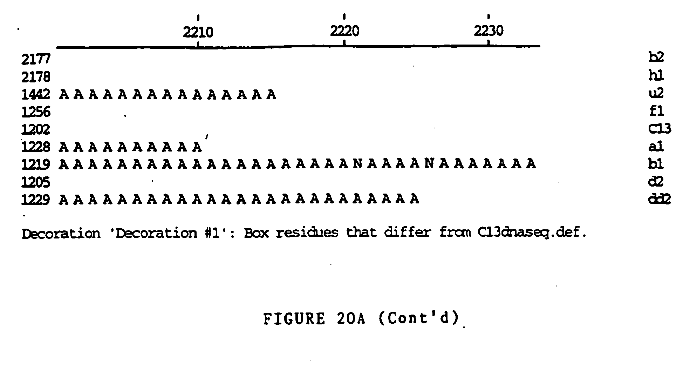

Figure 20A shows the nucleotide sequences of CL13 variants (a1, b1, b2, d2, dd2, f1, u2 and h1) and the original CL13 (denoted C13).

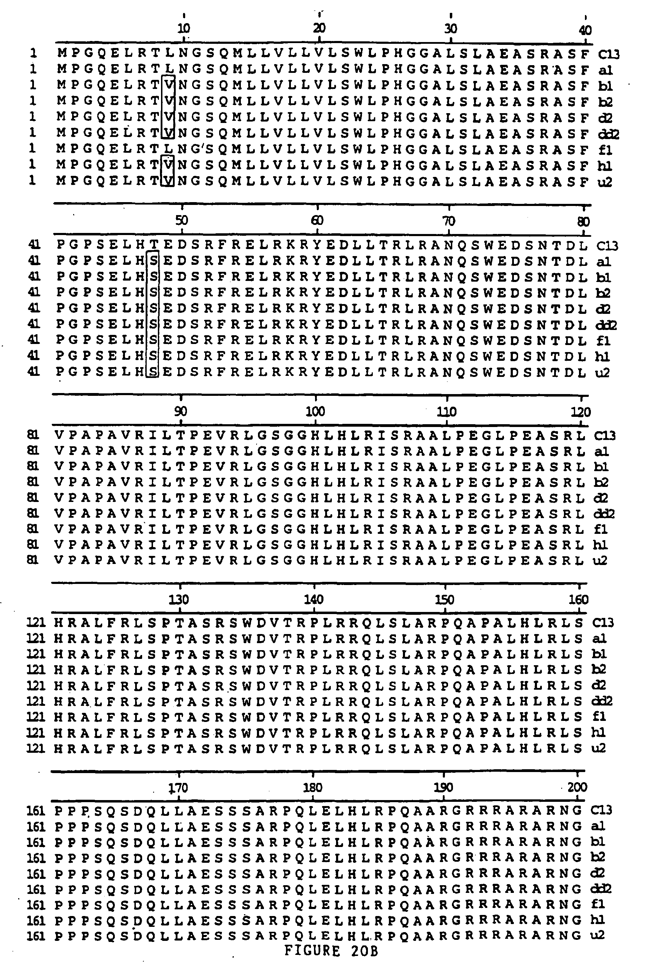

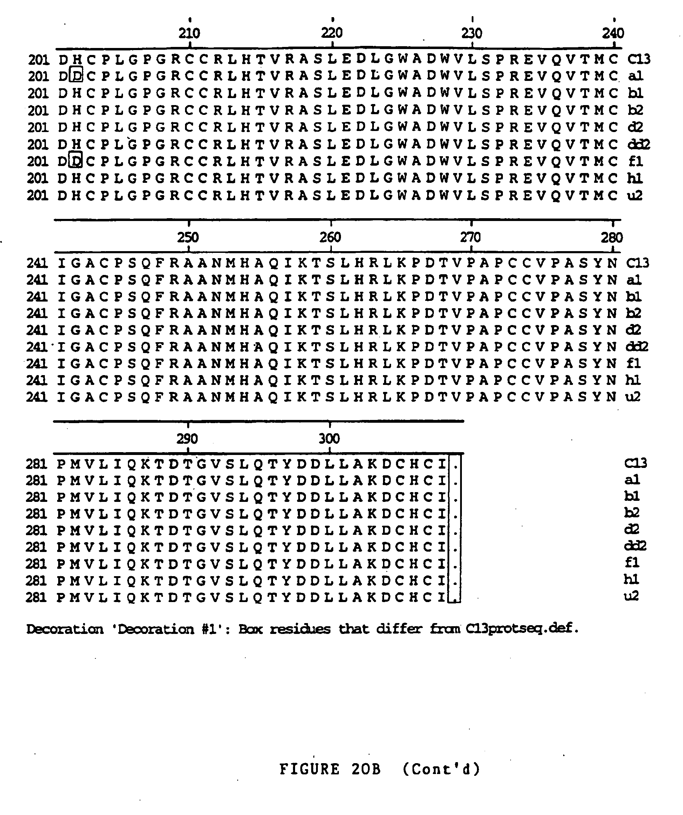

Figure 20B shows a comparison of a portion of the putative amino acid sequence of the CL13 variants a1, b1, b2, d2, dd2, f1, u2 and h1.

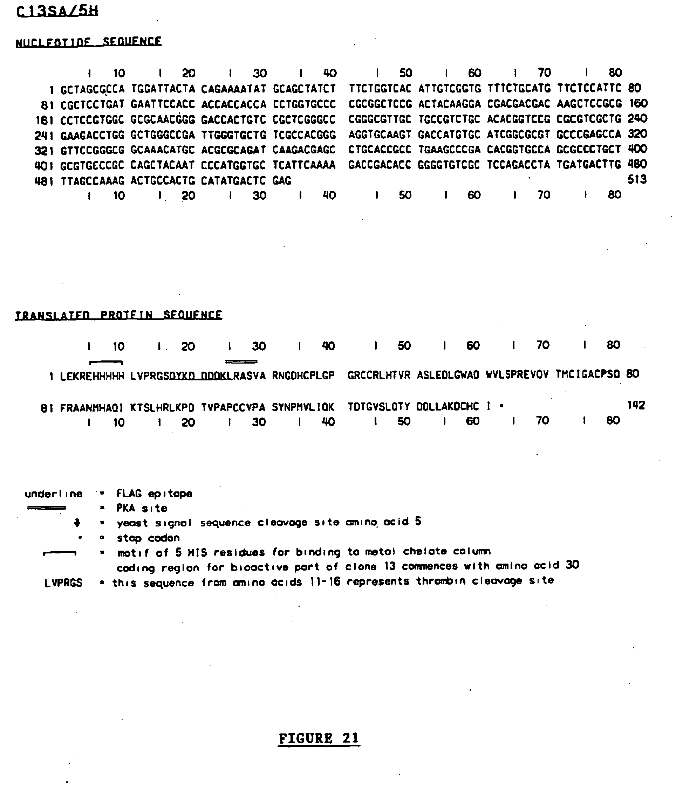

Figure 21 provides the nucleotide sequence and putative amino acid sequence for clone 13 in construct C13SA/5H (HIS Thrombin cleavage site-FLAG-PKA-mature bioactive CL13 peptide). This construct has been used for expression in the yeast *Pichia pastoris.* HIS is 5 histadine residue motif to allow affinity purification using Nickel chelate chromatography. The thrombin site is to allow enzymic cleavage of the HIS from the rest of the sequence if required.

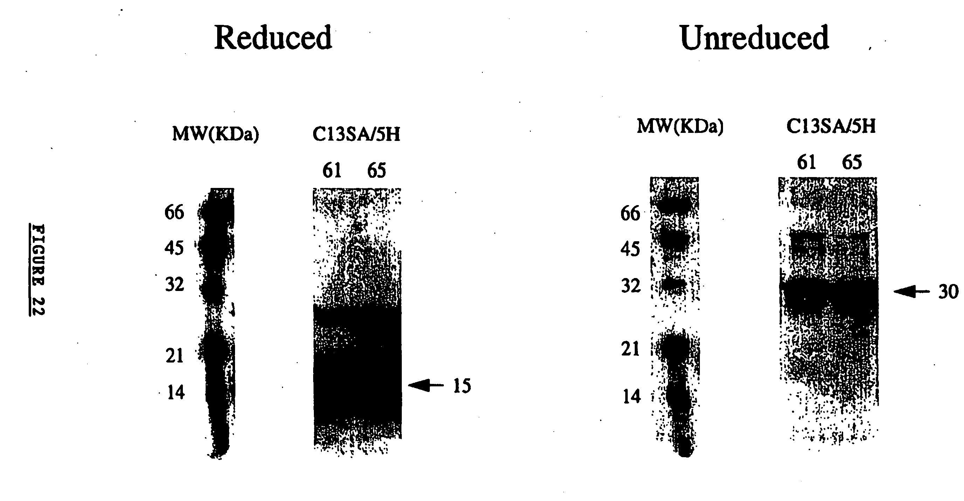

Figure 22 shows a western blot of culture medium from *Pichia pastoris* transformed with construct C13SA/5H. pCL13 protein is visualised using anti-FLAG antibody.

### EXAMPLE 1: Characterisation of Clone 13

This clone hybridizes on Northern blot to a single species of mRNA of 1.2kb size and the gene has been localised by fluorescent *in situ* hybridisation to chromosome 19p13.1 (TGF-b1 is on 19q13.1 and MIS is on 19p13.3). The characteristics of the clone are outlined below.

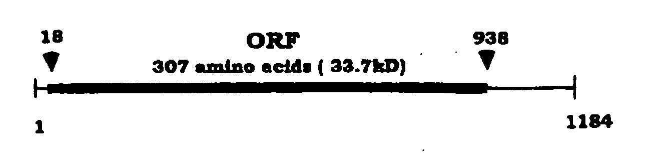

The largest open reading frame codes for a high cysteine containing protein with a signal peptide. It bears strong homology to members of the TGF-β superfamily (including TGF-b itself) when analysed using the *fasta* program on the ANGIS facility (opt scores 180-250). Extensive multiple sequence alignment using the CLUSTAL V program on GCG has been undertaken with the CL13 translated amino acid sequence (pCL13) and most members of this superfamily (see Figure 2).

Mature pCL13 is a dimeric protein with a conserved RXXR site that is likely to be involved in cleavage of a large pro-peptide with the encoded polypeptide decreasing from a predicted monomeric mass of about 34kDa to 13kDa. pCl13 has a potential glycosylation sites in its pro-peptide, but none in the mature protein, suggesting that glycosylation may be for intracellular targeting as it is in TGF-β.

In this superfamily the bio-activity resides in the carboxy terminal half of the molecule. There is strong conservation in this region between all superfamily members, especially in 7 of the cysteine residues. The full alignment data unequivocally demonstrates that pCL13 belongs to the TGF-β superfamily. In this superfamily within family identity is of the order of 70-80%. pCL13 does not display identity of this degree to any of the individual families and therefore appears to represent an entirely new and separate category within the TGF-β superfamily.

The full nucleotide sequence and putative amino acid sequence for clone 13 (CL13) is provided at Figure 1.

### EXAMPLE 2: CL13 Gene Expression and Analysis of Biological Activity

Extensive studies of regulation of CL13 gene expression have been undertaken using the ³²P labelled clone insert and the results are summarised at Table 1. Some examples are also illustrated in Figures 2, 3 and 4. The results indicate that the 1.2 kb transcript was present at very low levels in untreated U937 and culture derived macrophages. Expression increased markedly with phorbol 12-myristate 13-acetate (PMA), but was not upregulated by LPS or interferon-g (IFN-g). Clone 13 was expressed strongly in macrophages treated with GM-CSF, M-CSF, IL2 or TNF-α and to a lesser extent with TGF-β, PDGF-BB or IL-6. There was also increased expression of CL13 mRNA in a human neonatal fibroblast cell line (CCD34Lu) in response to IGF-1, PDGF BB, TGF-β or TNF-α and in human umbilical vein endothelial cells grown with ECGF. No expression of this gene was found in either resting or activated B or T lymphocytes/cell lines.

We can deduce reasonable hypotheses about the nature of the biological role of this protein on the basis of its expression and the general characteristics of the superfamily. CL13 expression could be induced in culture derived macrophages (MAC) by a variety of activation agents including cytokines and PMA but not LPS. Its expression was also induced in fibroblasts by activation and could not be induced at all in lymphocytes. As the endothelial cells tested were grown in the presence of ECGS, it is not possible to conclude whether expression is absent under resting conditions.

It may be of particular significance that TGF-β induces expression of CL13 in both fibroblasts and MAC. It is possible that some of the functions ascribed to TGF-β may be due to an autocrine or paracrine induction of TGF-β by CL13.

Many of the proteins in this TGF-β superfamily act on mesenchymal cells and it is anticipated that this will be true for pCL13. It is also thought that pCL13 may enhance the effector function of these cells, perhaps in a manner similar to TGF-β itself.

Lymphocytes and macrophages are intimately related in biological function. The fact that lymphocytes do not appear able to express CL13, but MAC express it in large amounts suggests the possibility that the lymphocyte may also represent a target for pCL13 .

In summary, pCL13's properties and pattern of expression suggest that there may be some similarities to TGF-β. However, whilst it belongs to this superfamily, it can be said with some certainty, on the basis of sequence comparison, that pCL13 is one of a new class of proteins within this superfamily and is not an undescribed TGF-β protein (e.g. TGF-β6).

**TABLE 1**

| **SUMMARY OF NORTHERN BLOT ANALYSIS OF CLONE 13^{#}** | | |
|---|---|---|
| | **TREATMENT** | **1.2 kb RNA** |
| Monocytoid cell lines: HL60, KG1 | untreated | - |
| | RA or PMA | - |
| | RA/PMA | + |
| Monocytoid cell line: U937 | untreated | + |
| | IFNg or LPS or both | + |
| | RA alone or with LPS | ++ |
| | PMA | +++ |
| | RA/PMA (3 h) | ++++ |
| | RA/PMA (12 h) | +++++ |
| | TGFb | ++ |
| | PMA/IL4 | ++ |
| Macrophages¹ | untreated | - |
| (peripheral blood derived) | RA | + |
| | PMA | +++ |
| | RA/PMA | ++++ |
| | LPS or IFN-g | - |
| | IFN-g followed by IL2 | + |
| | GM CSF | + |
| | IL 6 or IL2 or PDGF BB or TGF b | ++ |
| | M CSF or IL1 b or TNFa | +++ |
| B cell lines², T cell lines. | | |
| peripheral blood T cells | with or without PMA | - |
| Fibroblasts (CCD 34 Lu) | nil | - |
| | cytokines ³ | + |
| Replicating endothelial cells⁴ | with or without cytokines⁵ | + |

| | | |
|---|---|---|
| #Standardisation of the blots was achieved by probing with an oligonucleotide for 28S rRNA; All cell lines are human : 1. Macrophages are serum-free. 2. B cell lines were Sultan. Daudi. RPMI and U266. 3. Cytokines were IGF1, PDGF BB, TGFb and TNFa for hrs. 4. HUVEC was grown with 20% FCS & growth factor (ECGF). 5. Cytokines were IFNg. TNFa. IL1b and IL2 for 3h. | | |

### EXAMPLE 3: Expression of Recombinant pCL13 and Antibody Generation

### 1. Prokaryotic expression of CL13

This-has been undertaken using the pGEX vector which generates a glutathione-S-taansferase fusion protein. Material of the correct molecular weight was synthesised but was denatured and insoluble and hence unsuitable for purification. As a consequence, no further work was done with this vector because of the difficulties that are likely to be involved.

### 2. Eukaryotic expression of CL13

### General Approach

A number of DNA constructs based on the CL13 have been made. To some of these constructs the DNA sequence for the FLAG epitope has been added. This epitope codes for the 8 amino acid peptide (N-Asp-Tyr-Lys-Asp-Asp-Asp-Asp-Lys-C) which codes for an enterokinase cleavage site is recognised by two commercially available monoclonal antibodies. A protein containing this marker peptide can then be affinity purified using these antibodies. Additionally the protein can also be detected using Western blotting or other antibody based assays. Addition of this small hydrophilic peptide of the amino terminal region of the construct would not be expected to influence the bioactivity of the whole protein. However, if desired, enterokinase can be used to selectively cleave the FLAG peptide from the construction, without affecting the rest of the molecule.

Prediction of the signal sequence cleavage site of any protein is only 75-80% accurate. For this reason in some constructions it was necessary to use the follicle stimulating hormone (FSH) leader sequence. It is known to function in the eukaryotic cell to be used for transfection and its precise cleavage site is known. This was important to ensure that the FLAG peptide remained attached to the propeptide and was not removed with signal sequence cleavage.

The following DNA constructs were made:
1. CL13: Unmodified full C13 sequence (Figure 1). (CL13 leader sequence-Sequence for CL13 propeptide-Sequence for mature bioactive CL13 peptide).
2. C13LB: Full length CL13 with FLAG (Figure 6). (FSH Leader sequence-FLAG-CL-13 propeptide-Sequence for mature bioactive CL13 peptide).
3. FFC13S: Bioactive CL13 with FLAG (Figure 7) (FSH Leader sequence-FLAG sequence-about 40 amino acids propeptide-Sequence for mature bioactive CL13 peptide).
4. C13SA: Bioactive CL13 with FLAG (Figure 8) (FSH leader - FLAG sequence - PKA - Mature bioactive CL13). PKA is the recognition sequence for protein kinase A to allow *in vitro* phosphorylation.

These constructs were cloned into two different mammalian cell expression vectors. These are the pCEP4 vector which is a semipermanent expression vector or the pCEP4 vector from which the EBNA gene sequence has been deleted to allow it to permanently integration into the genome of the mammalian cell into which it is transfected. This allows for the development of a permanent cell line secreting this protein. The constructions have all been transferred into CHO and COS cells and either semipermanent or permanent cell lines bearing the transfectant established with the use of hygromycin to kill non transfectant bearing cells. Protein production and purification have been undertaken to date only in construction numbers 2, 3 and 4 (dominantly 3 and 4), bioactive CL13 with FLAG.

### Cell Culture

Both COS and CHO cells are grown in Ham's F12 medium with 5% foetal calf serum (FCS) and 400ug/ml hygromycin (only in semipermanent cell lines). At confluence, medium is removed and replaced with HamF12 containing no serum or other supplements. The conditioned medium is removed after 3 days and used for purification of recombinant FLAG-CL13. The cells are then passaged and once more placed in serum containing medium.

### Quantification

A dot blot assay has been established for quantification of recombinant FLAG containing proteins - either in culture supernatant or in purified form. Protein from culture supernatants (10-100ul) is deposited onto nitrocellulose using a dot-blot apparatus. The membrane is then reacted with monoclonal anti-FLAG antibody and then biotinylated rabbit anti mouse IgG. This is then visualised by enhanced chemilumininescence on autoradiographic film. A standard curve is generated using a protein bacterial alkaline phosphatase (BAP) that has been engineered so that it contains 1 copy of the FLAG epitope at its amino terminus (Mr 50-55 kDa). The sensitivity of this assay is about 20ng of BAP.

When this assay was used to analyse the production of FLAG-CL13 it was found that cultures produced between about 25 and 400ng of recombinant protein per ml of culture supernatant. The best expression is seen with constructs 3 and 4.

### Purification

Recombinant protein containing medium is incubated with sepharose beads to which anti-FLAG antibody has been conjugated. Approximately 1 ml of beads is used per 100 ml of conditioned medium. The sepharose and medium are incubated for 18hrs at 3deg C then beads are pelleted and poured into a minicolumn. They are then washed extensively with PBS and the recombinant protein is released with FLAG peptide. This is a very gentle but efficient procedure and ensures that the bioactivity of the recombinant protein is not damaged. The FLAG peptide is removed using gel filtration chromatography. The beads are then stripped with pH 3.5 glycine buffer and can then be re-used.

Figure 9 shows a Western blot of purified pCL13 protein from C13LB and C13SA constructs. The purified material was electrophoresed using SDS PAGE on a 15% gel under reducing and non reducing conditions prior to Western blotting and visualisation using monoclonal anti-FLAG antibody. The constructs migrate at molecular weights slightly higher than predicted, something that seems to be a function of the amino acids in the FLAG sequence and has been previously reported with the use of this epitope tag. However, there is the expected change in molecular weight associated with the use of reducing conditions indicating that the material is in the dimeric conformation.

The fact that these dimeric proteins are secreted into the medium also indicates that they are folded correctly as improperly folded and aggregated proteins expressed in eukaryotic cells are not secreted. The two constructs (FFC13SC and C13SC) which encode the bioactive protein alone, both appear to be expressed at much higher levels than the native CL13 sequence which has only been modified to contain a FLAG epitope (C13LB). This is exemplified in Figure 9 which compares relative protein expression from constructs C13SA and C13LB.

### EXAMPLE 4: Effect of pCL13 on Fibroblast Function

TGF-β stimulates fibroblast differentiated function and inhibits replication. In order to compare the function of pCL13 with TGF-β, the effect of pCL13 on fibroblast functions may be examined as follows.

### a. Collagen and Glycosaminoglycan production

Neonatal lung fibroblasts (CCD34LU) can be grown to confluence and the growth medium replaced with DMEM. containing 0.1% BSA. The cells can then be stimulated with recombinant pCL13 or TGF-β (10ng/ml) as a positive control. The culture supernatants can then be collected 18 hours later and assayed for total collagen and glycosaminoglycans (GAG). Collagen synthesis can be measured using a microtitre plate colorimetric assay developed in this laboratory which depends on the binding of total collagen to the dye sirius red (18). Total sulphated GAG can be measured with a colorimetric assay adapted in this laboratory for microtitre plate format and which has already been used for the *in vitro* determination of fibroblasts GAG synthesis (9,10). This assay is based on the metachromatic shift in absorption maximum for the cationic dye dimethyl-methylene blue consequent on binding the polyionic moieties of GAG (9,10).

### b. Fibroblast replication

TGF-β is known to have a variable effect on in vitro fibroblast proliferation that probably depends on the balance between its capacity to down-regulate the PDGF receptor and the its induction of fibroblast PDGF synthesis. To determine whether pCL13 also modifies replication, a growth factor assay will be undertaken with CCD34Lu essentially as previously described (11, 12, 13)). These cells are sparsely plated at a concentration of about 1000 cells/ well ( 96 well plate). pCL13 protein or TGF-β (100ng/ml) (positive control) will be either added alone or in combination with a known fibroblast growth factor present within fetal calf serum. Growth factor activity can be determined by ³H-thymidine incorporation.

### c. Collagenase activity

It would be expected that pCL13 inhibits the induction of collagenase activity. To test this, neonatal lung fibroblasts (CCD34LU) can be grown to confluence then the growth medium replaced with DMEM containing 0.1% BSA. The cells can then be stimulated with recombinant PMA to induce synthesis of collagenase in either the presence or absence of pCL13 or TGF-β (50ng/ml - positive control). The supernatants can then be assayed for collagenase activity using our adaptation (14) of an assay (15) that is based on the degradation of 20ug of purified type I collagen that has been coated onto a microtitre plate. The undigested collagen is visualized by staining with sirius red and quantified photometrically.

### EXAMPLE 5: Effect of pCL13 on Macrophage Function

The effects of TGF-b on macrophages are complex and in some instances apparently paradoxical. In general terms TGF-b has been considered to be a potent macrophage chemotactic agent, a down-regulator of macrophage activation and a promoter of differentiation (3,4). To test the effect of pCL13 on macrophages, culture derived macrophages (MAC) will be used as the major cell source and will be grown free of serum in Iscove's Modified Dulbecco's Medium, using methods established by our laboratory (15,16). As replicating cells become non adherent, it is possible to utilise both adherent, and undamaged non-adherent MAC for study.

### a. Chemotaxis

This may be examined using a standard Boyden chamber chemotaxis assay as previously performed (17). TGF-β (1pg/ml) will be used as the positive control for chemotaxis, and its response will be compared with that of pCL13.

### b. Monocytoid cell differentiation

Both PMA and retinoic acid (RA) are potent inducers of CL13 mRNA. Both PMA, RA (as well as TGF-β) are known to induce the *in vitro* differentiation of the primitive human monocytoid cell lines U937 and HL60 as well as bone marrow monocyte precursors. To examine the role of pCL13 in this process, the U937 and HL60 cell lines can be grown in the presence of TGF-β, pCL13 (with or without additional RA). Their differentiation will be monitored by morphology, increased adherence and inhibition of replication (³H-thymidine incorporation).

It has been previously demonstrated that human MAC grow in serum free medium, and their differentiation from monocytes to macrophages in vitro can be monitored by the expression of surface CD71, the transferrin receptor (13,15). This is not seen on the surface of monocytes but is found on most MAC by day 7 of culture. Cells will be grown with TGF-b or pCL13 or interferon gamma then stained with fluoresceinated CD71 antibody and examined flow cytometrically on day 3 of culture (13). Promotion of differentiation will be associated with earlier expression of this surface antigen.

### c. Cytokine production

TGF-β has been reported to inhibit LPS induced production of TNF-α and IL-1. Further, as TGF-β induces pCL13 expression in a number of situations, it is possible that some of the functions ascribed to TGF-β may be contributed to by pCL13. This can be examined using the above bioassays in which both TGF-β and pCL13 are active. The fibroblasts will be stimulated by TGF-β in the presence of blocking pCL13 antibody and pCL13 in the presence of a blocking TGF-β antibody. If autocrine pathways are in operation, the function in question should be reduced or inhibited by the blocking antibody. Antisense oligonucleotide inhibition experiments can also be undertaken.

### EXAMPLE 6: Effect of pCL13 on Endothelial Cells

Like TGF-β, pCL13 may modify endothelial expression of adhesion molecules with subsequent downregulation of adhesion of neutrophils, monocytes or lymphocytes. Additionally pCL13 may modify angiogenesis and endothelial mediator production. This may be investigated by investigating the effect of pCL13 on:
(i) Leukocyte adherence to resting and cytokine activated vascular endothelium;
(ii) Endothelial production of cytokines such as IL-8, MCP-1, IL-.1, IL-6, and endothelin;
(iii) Endothelial prostanoid synthesis;
(iv) Endothelial procoagulant activity; and
(v) Angiogenesis (in vitro and vivo).

### EXAMPLE 7: Effect of pCL13 on Lymphocyte Function

Like TGF-β, pCL13 may act as an immunosuppressive agent. This can be investigated by determining the effect of pCL13 on:
(i) T and B cell proliferation;
(ii) Immunoglobulin synthesis;
(iii) LAK cell and NK cell activity; and
(iv) Production in vitro of cytokines (protein and/or mRNA) such as IL-2, IFN-g, IL-4, IL-5, IL-10.

### EXAMPLE 8: Effect of pCL13 on Tumor Cell Proliferation

pCL13 may like TGF-β inhibit tumor cell replication and promote tumor differentiation. This can be investigated by determining the effect of pCL13 on:
(i) In vitro investigation of the proliferation of a wide range of tumour cell lines available through the ATCC; and
(ii) Observing change in tumor phenotype towards a more differentiated form (e.g. change from non-adherent to adherent phenotype).

### EXAMPLE 9: Effect of pCL13 on Glycosaminoglycan Production by Non-transfected and transfected CHO cells.

The effect of pCL13 on glycosaminoglycan production was investigated using non-transfected and transfected CHO cells. Figure 10 shows glycosaminoglycan analysis in the non-transfected (KI) and CL13 transfected (P4N, 15 and 24) CHO cells using dimethyl-methylene blue (DMB) assay (9, 10). P4N, 15 and 24 produce increasing amounts of pCL13 respectively. Cells were cultured in DMEM/F-12 medium containing 5% FCS for 5 days. Cells were then changed to FCS-free DMEM for 24 hours. To 100 µl cell culture medium 100 µl DMB dye was added and the absorbance was read at 492 nm immediately. The results represent the mean +/- SD of triplicate wells.

### EXAMPLE 10: Effect of pCL13 on Collagen Production by Non-transfected and Transfected CHO Cells

The effect of pCL13 on collagen production was investigated. Figure 11 shows the effect of pCL13 on collagen production by non-transfected (K1) and CL13 transfected (P4N, 15 and 24) CHO cells. P4N, 15 and 24 produce increasing amounts of pCL13 respectively. Cells were cultured in DMEM/F-12 medium containing 5% FCS for 5 days. Cells were then changed to FCS-free DMEM for 24 hours. The amount of collagen produced by these cells was determined using a Biodot apparatus. Culture supernatant (50 µl) was placed on nitrocellulose membrane. The membrane was washed in 100 µl PBS and dried. Collagen retained in the nitrocellulose membrane was stained with 0.1% Sirius red dye (18). Individual spots were cut out and eluted with 0.1 N NaOH and absorbance was read at 550 nm. The results represent the mean +/- SD of triplicate wells.

Examples 11 to 16 described hereinafter were conducted with the C13SA construct or pCL13 produced from the C13SA construct. As described above, the C13SA construct varies from CL13 in that it includes no propeptide encoding sequences.

### EXAMPLE 11: Effects of pCL13 on matrix protein production

### a. Glycosaminoglycan production

Figure 12 shows the effect of pCL13 on 35S-labelled-proteoglycan production in 3T3 (mouse fibroblasts) and neonatal human lung fibroblasts (CCD 34 Lu) after 24 hour incubation. Confluent cells were changed to RPMI culture medium containing 0.1% BSA and 50µg/ml ascorbic acid for 24 hours. Cells were then incubated with different pCL13 concentrations in the presence of 10µlCi/ml [³⁵S] sulphate for 24 hours. At the end of the incubation period medium was removed and protease inhibitors were added. Proteoglycans present in the extracellular matrix were extracted using 4M guanidine hydrochloride containing protease inhibitors for one hour at 4°C. Total proteoglycan production in the medium and the cell fraction was determined using Sephadex G-25 chromatography columns (19). The results represent the mean +/-SD of triplicate wells.

In 3T3 cells, after 24 hour incubation period, pCL13 caused a dose dependent increase in the proteoglycan production. A 92% increase was observed at 25ng/ml pCL13 concentrations and 60% increase was seen at 6.7 and 2.2 ng/ml pCL13 concentration. In comparison TGF-β at 20ng/ml elevated proteoglycan production by 95%. In CCD 34Lu cells, pCL13 at 50ng/ml caused 23% increase in the proteoglycan production and 6% increase at 25ng/ml pCL13 concentration. In comparison TGF-β at 10ng/ml elevated proteoglycan production by 36%.

### b. Collagen production

Figure 13 shows the effect of pCL13 on collagen type 1 production in neonatal lung fibroblasts (CCD 34 Lu). Confluent cells were changed to DMEM culture medium containing 0.1% BSA and 50µg/ml ascorbic acid for 24 hours. Cells were then incubated with different pCL13 concentrations in the presence of 50µg/ml b-aminopropionitrile for 24 hours. At the end of incubation period the amount of collagen present in the medium was determined using an ELISA. Briefly, supernatants from treated and non-treated fibroblasts as well as type 1 collagen standards were incubated for 72 hours at 4°C in 96-well microtitre plates (NUNC). At the end of incubation period plates were washed, blocked with 4% bovine serum albumin in phosphate buffered saline, incubated with collagen type 1 monoclonal antibody (Sigma). The plates were then rewashed and biotinylated mouse IgG was added and followed by streptavidin complex. After the addition of substrate, plates were read at 490/405 nm on a plate reader. The results represent the mean +/-SD of triplicate wells. pCL13 at 50ng/ml caused 140% increase in the collagen production, 190% increase at 25ng/ml and 11% at 5ng/ml concentration. In comparison TGF-β at 10ng/ml elevated collagen production by 34% after 24 hours. The relatively poor TGF-β response has occurred because TGF-β requires 48-78 hours to achieve maximal effect.

### EXAMPLE 12: Effect of pCL13 on fibroblast replication

### a. Growth under limiting serum conditions

The growth factor activity of pCL13 and transforming growth factor beta (TGFβ) on growth-arrested BFF (human baby foreskin fibroblasts) and 3T3 (mouse fibroblasts) cells was determined. The cytokines were added to BFF and 3T3 cells in 0.2% foetal bovine serum (FBS) media to determine whether they were true growth factors which could stimulate a resting cell to progress through the cell cycle and undergo division. The growth factor assay was performed as previously described (11, 12). In brief, the cells were plated at 1.2x10³ cells/well in 200mL of growth-arresting medium (0.2% FBS) for 72h. The media was then replaced with fresh 0.2% FBS media with or without cytokines and 0.5mCi/well of [3-H] Thymidine for a further 72h. The cells were then harvested with an automated cell harvester and the thymidine uptake into proliferating cells was counted on a liquid scintillation analyser. The controls included 0.2% FBS media only, 10% FBS media only (normal growth media), and pCL13 diluent (0.1% CHAPS) in 0.2% FBS media.

The results shown in Figure 14 indicate that pCL13 appears to have true growth factor activity on both human BFF. TGFβ appears to be inhibitory for BFF cells. Neither pCL13 not TGFβ exhibit growth factor activity on 3T3 under the conditions of this assay.

### b. Growth in the presence of serum

The growth factor activity of pCL13 and transforming growth factor beta (TGFβ) on growth-arrested BFF (human baby foreskin fibroblasts) and 3T3 (mouse fibroblasts) cells was determined. The cytokines were added to BFF and 3T3 cells in 2% foetal bovine serum (FBS) media to determine whether they were growth enhancing substances which could enhance the rate at which the cells moved through the cell cycle. The growth factor assay was performed as previously described (11, 12). In brief, the cells were plated at 1.2x10³ cells/well in 200ml of growth-arresting medium (0.2% FBS) for 72h. The media was then replaced with fresh 2% FBS media with or without cytokines and 0.5mCi/well of [3-H] Thymidine for a further 72h. The cells were then harvested with an automated cell harvester and the thymidine uptake into proliferating cells was counted on a liquid scintillation analyser. The controls included 2% FBS media only, 10% FBS media only (normal growth media), and pCL13 diluent (0.1% CHAPS) in 0.2% FBS media.

The results (Figure 15) show that pCL13 had a growth-enhancing effect on human BFF and murine 3T3 cells. TGFβ appears to be inhibitory for BFF cells but to have growth-enhancing activity at low concentration on 3T3 cells.

### EXAMPLE 13: Effects of pCL13 on replication of human monocytoid cell lines

pCL13 was compared with transforming growth factor beta (TGFb) and interferon alpha 2b (IFNa2b), for their antiproliferative effect on the cell line U937 (a human monocyte-like histiocytic lymphoma) and Mono Mac 6 (a monoblastic leukemia cell line). The cells were plated at 3x10⁴ cells/well in 200mL of 10% FBS (foetal bovine serum) medium with or without cytokines. For the final 6h of a 48h incubation period, the wells were pulsed with 0.5mCi/well of [3-h] Thymidine. The cells were then harvested with an automated cell harvester and the thymidine uptake into proliferating cells were counted on a liquid scintillation analyser. The controls include 10% FBS medium alone and pCL13 diluent in 10% FBS medium.

The results (Figure 16) indicate that two batches of pCL13, B447 and B448A. at concentrations of 10 and 100ng/ml have a small antiproliferative effect on two human cell lines of monocytic origin. This contrasts with the stronger antiproliferative effects of TGFβ (2 and 20ng/ml) and IFNa2b (10³ and 10⁵ U/ml) on U937 and Mono Mac 6 cells.

### EXAMPLE 14: Effects of pCL13 on macrophage production of TNF

The data in Figure 17 shows the effect of different pCL13 concentration on LPS stimulated TNF-α production from human culture derived macrophages. Monocytes were purified by elutriation from buffy coats and cultured in Iscove's medium containing 0.1% BSA (13, 16). On day 5, cells were incubated with different pCL13 concentrations in the presence of 10µg/ml LPS in the Iscove's medium for 24 hours. At the end of incubation period medium was removed and the amount of TNF-α present was determined using a sandwich ELISA (Genzyme). The results show that pCL13 caused inhibition of LPS induced TNF-α production. A 47% inhibition was observed at 20ng/ml pCL13 and a 27% inhibition was seen at 7ng/ml of pCL13. In comparison TGF-β only brought about 10% reduction at 20ng/ml.

### EXAMPLE 15: Effects of pCL13 on tumor cytotoxicity

The direct effect of pCL13 and TGFβ on tumour target cells (5637 bladder carcinoma and MDA-MB-231 breast adenocarcinoma) and the effect of pCL13 and TGFβ on monocyte-mediated killing of tumor cells was examined by measuring the release of radiolabelled DNA from lysed tumour target cells. The cytotoxicity assay was performed as previously described (20). Tumour target cells (labelled while in the exponential growth phase with 20µCi of [³H] Thymidine/1x10⁶ cells for 24h) were added to the monocytes (effectors) at an effector:target (E:T) ratios of 10:1 for 72 h. The cells were then centrifuged and the supernatants counted in scintillation fluid on a liquid scintillation analyser. The controls included untreated tumour cells, untreated tumour cells co-cultured with monocytes and cytokine diluent alone. TGFβ and pCL13 were incubated with monocytes for 48h.

The results are shown at Figure 18. Neither pCL13 nor TGFβ had a direct cytotoxic effect on the 5637 or MDA-MB-231 tumour lines. However pCL13 enhanced the ability of monocytes to kill 5637 cells. pCL13 also enhanced the monocyte-mediated killing of T24 (bladder carcinoma), J82 (bladder carcinoma), T47D (breast ductal carcinoma) and JCPL (ovarian carcinoma)(data not shown).

### EXAMPLE 16: In vivo Effects of pCL13

Rats (Fisher F343) were injected subcutaneously on their backs with 0.1ml of three concentrations of pCL13, a negative saline control and TGFβ. The injections were widely separated and each animal was administered with the whole panel of 5 injections. The amounts of pCL13 injected were 60ng. 30ng and 2ng. The dose of TGFβ administered was 10ng. The animals were then sacrificed at intervals commencing at 3 hours and up to 2 week following administration. Three animals were used for each time point, and following sacrifice the areas in which material had been administered was excised, formalin fixed, mounted, then stained with haematoxylin and eosin. The material was then evaluated microscopically.

### a. Macroscopic Changes

There was no macroscopic difference between the biopsies in any of the animals, under any of the various conditions other than at the two week time point. At the two week time point however, the biopsies, only of the areas with the two highest doses of pCL13, showed obvious macroscopic differences in the area between the muscle and skin. This area seemed somewhat expanded and had a white glistening appearance, suggestive of excess matrix protein deposition.

### b. Microscopic Evaluation

No differences were seen on histological sections at the three hour time points. However at the day one (24 hour) time point the areas in which the two highest concentrations of pCL13 had been administered demonstrated a mononuclear cell infiltrate which was somewhat patchy in character and was present dominantly in the subcutaneous tissue (Figure 19A). No similar changes were observed in either the negative saline control (Figure 19B) or TGFβ at a dose of 10ng/ml. The infiltrate seemed to be present maximally at days one and two and be markedly diminished or absent from day four onwards. These findings suggest that pCL13 was chemotactic for macrophages and or lymphocytes.

This study was not undertaken in such a manner as to be able to supply good quantitative data on the amount of collagen that was present in the areas where the two substances were administered. However in conjunction with the macroscopic appearance, it appears likely that the amount of collagen was increased in the samples containing the two highest doses of clone 13, at least at the two week time point.

### EXAMPLE 17: Clone 13 Variants

Re-screening was undertaken using a fetal lung cDNA library using a portion of the coding sequence of clone 13 as a probe. This was undertaken in order to determine the existence of clone 13 variants. Using this approach a number of additional clones (a1 b2, h1, b1, d2, dd2, f1 and u2) were obtained and the sequence of these clones is illustrated in Figure 20A which shows the nucleotide sequence and Figure 20B which shows a portion of the translated open reading frame. It also compares the sequences with that of the original clone 13 sequence (C13). From Figure 20B, it can be seen that the translated coding region of these clone 13 variants displays only minor differences. These occur at amino acids 9, 48 and 202. These are all in the propeptide region and are likely to represent genetic differences between the individuals whose RNA was used to prepare the cDNA library. However, at the DNA level, there is substantial variation dominantly in the 5' untranslated region, but to a lesser extent in the 3' untranslated region. Whilst these variants may well be important in areas such as transcriptional regulation they are untranslated and hence cannot affect bioactivity.

Some of the clones isolated and displayed in Figure 20A (e.g. b2 and h1) even though they have very long 5' untranslated region, still do not represent the complete coding sequence. This can be ascertained as when the 5' untranslated region is used as a probe, on northern blots, hybridisation to a band of approximately 7kb is demonstrable. The reasons for this marked length variation are unclear but could include alternate splicing of an untranslated exon, the use of alternate transcriptional start sites or even gene duplication.

### EXAMPLE 18: Expression of clone is using a yeast eukaryotic system

The bioactive region of clone 13, modified at its amino terminus so as to contain a number of additional marker epitopes (construct C13SA/5H - Figure 21) was cloned into the pPIC9 plasmid. This plasmid was then used to transform the yeast *Pichia pastoris* according to the manufacturers instructions (Invitrogen Corp.). Yeast, successfully transformed by this plasmid were selected on the basis of methanol sensitivity. Colonies of yeast were then grown for two days, in suspension as per the manufacturers instructions. Culture medium was collected and an aliquot subjected to SDS-PAGE followed by western blotting. pCL13 containing bands were visualised using the anti-FLAG M2 antibody using standard procedures. Electrophoresis was carried out under both reducing and non-reducing conditions. It can be seen that large amounts of the protein are produced which are easily detectable with unconcentrated yeast culture medium, indicating secretion of the protein in an appropriate manner (Figure 22). The molecular weight approximates that expected on the basis of the amino acid composition and the doubling of the molecular weight under non-reducing conditions (Figure 22) indicates that the protein is, as expected, a disulphide bonded dimer. This is the correct structural configuration and indicates that the protein has been processed and secreted by the yeast organism in an appropriate manner.

The capacity to express this complex dimeric, protein with a high disulphide bond content in yeast systems is highly advantageous as it dramatically lowers the cost of production per unit quantity of protein and makes it far more suitable as a biopharmaceutical compared with material produced by mammalian cells.

Whilst this work has been undertaken with the yeast *Pichia pastoris,* it is quite likely that similar secretion will occur with a range of yeast organisms transduced with an appropriate yeast expression vector. As the bioactive region being expressed does not contain potential n-glycosylation sites, the hyperglycosylation, that sometimes occurs with mammalian proteins expressed by yeast strains, is not an issue.

### REFERENCES

1. Burt DW. Evolutionary grouping of the transforming growth-factor-b superfamily. Biochem Biophys Res Commun. 1992: 590-595.
2. Miyazono K, Ichijo H, Heldin C-H. Transforming growth factor-b: Latent forms, binding proteins and receptors. Growth factors 1993;8:11-22.
3. Wahl. SM. Transforming growth factor beta in inflammation: A cause and a cure. J Clin Immunol. 1992;12:61-74.
4. Roberts AB, Sporn MB. Physiological actions and clinical applications of transforming growth factor-b. Growth factors. 1993; 8:1-9.
5. Sambrook J., Fritsch EF. & Maniatis T. (1989) Molecular Cloning: A Laboratory Manual. Second Edition, Cold Spring Harbor Laboratory Press, New York.
6. Current Protocols in Molecular Biology, Ausubel FM etal, eds. Green Publishing Associates. New York. 1989
7. Ma J, Walsh B, Chen S-L, Penny R, Breit SN. Restoration of antibody activity of a monoclonal anti RNP antibody by HPLC under dissociative conditions: Demonstration of blocking of the antibody binding sites with antigen released from effete hybridoma cells. J Immunol methods. 1992: 155: 121-127.
8. Miyazono K, Thyber J, Heldin C-H. Retention of the TGF-b1 precursor in the golgi complex in a latent endoglycosidase H sensitive form. J Biol Chem. 1992; 267: 5668-75.
9. Farndale RW, Buttle DJ, Barrett AJ. Improved quantitation and discrimination of sulphated glycosaminoglcans by use of dimethylmethylene blue. Biochim Biophys Acta. 1986; 883: 173-177.
10. E.M Foulcher. MSc qualifying thesis submitted to UNSW entitled: Glycosaminoglycans and their role in inflammation. 1992.
11. Thornton SC, Por SB, Walsh B, Penny R, Breit SN. The interaction of immune and connective tissue cells: 1. The effect of lymphokines and monokines on fibroblast growth.J Leukocyte Biol. 1990; 47:312-320.
12. Thornton SC, Por SB, Shelley L, Penny R, Breit SN. Identification of the major fibroblast growth factors spontaneously released in inflammatory arthritis as PDGF and TNF alpha. Clin Exp Immunol. 1991; 86:79-86.
13. Bennett S, Por SB, Cooley MA, Breit SN. In vitro replication dynamics of human culture-derived macrophages in a long term serum free system. J Immunol. 1993: 150: 2364-2371.
14. Nethery A. Anal Biochem. 1986; 159: 390-395
15. Katelaris A. Doctor of Medicine thesis submitted to UNSW and entitled: Graft versus host disease as a model of scleroderma - An investigation into the interaction of the immune system and connective tissue using a murine model. 1991.
16. Bennett S, Por S, Stanley ER, Breit SN. Monocyte proliferation in cytokine-free, serum free system. J Immunol Methods. 1992;153:201-212.
17. Breit SN, Robinson JP, and Penny R. The effect of alpha 1 antitrypsin on phagocyte function. LLab Clin Immunol. 1983; 10:147-149.
18. Walsh B, Penny R, Breit SN. Microplate reader - based quantitation of collagens. Anal Biochem 1992; 203:187-190.
19. Bansal M.K., Ross A.S.A., and Bard J.B.L., Does chondroitin sulphate have a role to play in the morphogenesis of the check primary corneal stroma? Developmental Biology 1989; 133: 185-195.
20. Pryor K, J. Goddard, D. Goldstein, P. Stricker, P. Russell, D. Golovsky and R. Penny. Bacillus Calmette-Guerin (BCG) enhances monocyte- and lymphocyte-mediated blader tumour cell killing, British Journal of Cancer, 1995; 71: 801-807.

It will be appreciated by persons skilled in the art that numerous variations and/or modifications may be made to the invention as shown in the specific embodiments. The present embodiments are, therefore, to be considered in all respects as illustrative and not restrictive.

## Claims

1. An isolated polynucleotide obtainable from human chromosome 19p13.1, wherein said polynucleotide molecule comprises a nucleotide sequence encoding a protein belonging to the TGF-βsuperfamily having a conserved RXXR site and 7 conserved cysteine residues, wherein said encoded protein is capable of:
(i) being expressed by human neonatal fibroblast line CCD34Lu in increased levels in response to IGF-1, PDGF BB, TGF-β or TNF-α;
(ii) being expressed by macrophages in increased levels in response to GM-CSF, M-CSF, IL-2 or TNF-α; and
(iii) being expressed by human umbilical vein endothelial cells in increased levels in response to ECGF.

2. An isolated polynucleotide molecule according to claim 1, wherein the encoded protein is capable of:
(iv) exhibiting growth factor activity on human baby foreskin fibroblasts.

3. An isolated polynucleotide molecule according to claim 1 or 2, wherein said polynucleotide molecule comprises a nucleotide sequence corresponding to that shown in Figure 1.

4. An isolated polynucleotide molecule according to claim 1 or 2, wherein the nucleotide sequence corresponds to a variant nucleotide sequence selected from a1, b1, b2, d2, dd2, f1, h1 and u2 as shown in Figure 20A.

5. An isolated polynucleotide molecule according to any one of the preceding claims, wherein the nucleotide sequence encoding said protein does not comprise sequence encoding the native leader or propeptide.

6. An isolated polynucleotide molecule according to claim 5, wherein said nucleotide sequence encoding the protein includes sequence encoding a heterologous leader.

7. An isolated polynucleotide molecule according to claim 6, wherein said heterologous leader is the follicle stimulating hormone (FSH) leader.

8. A vector comprising a polynucleotide molecule according to any one of the preceding claims operably linked to a suitable promoter.

9. A vector comprising a nucleotide molecule according to claim 8, wherein the polynucleotide molecule is operably linked in opposite orientation to a suitable promoter such that expression proceeds 5' to the 3' terminus to produce antisense RNA.

10. A vector according to claim 9, wherein the polynucleotide molecule includes or is linked to a nucleotide sequence encoding a ribozyme domain.

11. A protein encoded by a polynucleotide molecule according to any one of the claims 1 to 7.

12. An organism transformed with a polynucleotide molecule according to any one of claims 1 to 7 or a vector according to any one of the claims 8 to 10, wherein said organism is selected from eukaryotic cell lines, yeast and plants.

13. An antibody or fragment thereof which specifically binds to the protein according to claim 11.

14. A protein or antigenic portion thereof, which binds to the antibody according to claim 13.

15. A method of producing a protein according to claim 11, comprising transforming a suitable host organism selected from eukaryotic cells lines, yeast and plants with a polynucleotide molecule according to any one of claims 1 to 7, wherein said polynucleotide molecule is constitutively or inducibly expressed in said host organism.

16. A pharmaceutical composition comprising a protein according to claim 11 or a protein or antigenic portion thereof according to claim 14 in admixture with a pharmaceutically acceptable carrier.

17. A protein according to claim 11 or a protein or antigenic portion thereof according to claim 14 for use in the treatment of a disease or condition selected from the group consisting of wound and/or fracture healing, ischaemic injury, cancer, autoimmune diseases, chronic inflammatory diseases, imunosuppresion, fibrotic/fibroproliferative disorders such as rheumatoid arthritis, artherosclerosis, pulmonary fibrosis, scleroderma, liver cirrhosis and keloids.

18. Use of a protein according to claim 11 or a protein or antigenic portion thereof according to claim 14 in the manufacture of a composition for the treatment of a disease or condition selected from the group consisting of wound and/or fracture healing, ischaemic injury, cancer, autoimmune diseases, chronic inflammatory diseases, imunosuppresion, fibrotic/fibroproliferative disorders such as rheumatoid arthritis, artherosclerosis, pulmonary fibrosis, scleroderma, liver cirrhosis and keloids.

19. A method of producing a protein according to claim 15, wherein said eukaryotic cell line is a mammalian cell line.

20. An in vitro method for diagnosing a disease or disorder in a subject, said disease or disorder being selected from cancer and inflammatory and fibrotic disorders, said method comprising detecting the presence of a protein according to claim 11.

21. The method according to claim 20, wherein the inflammatory and fibrotic disorder is rheumatoid arthritis, cirrhosis or atherosclerosis.

22. A kit for use in the method according to claim 20 or 21, wherein the kit comprises the protein according to claim 11 and/or the antibody or fragment thereof according to claim 13.

23. A kit for use in the method according to claim 20 or 21, wherein the kit comprises nucleotide primers for the nucleotide sequence according to any one of claims 1 to 7 for PCR based assays.

24. A protein according to claim 11 wherein the protein is the mature protein.

## Patentansprüche

1. Isoliertes Polynukleotid, welches von dem menschlichen Chromosom 19p13.1 erhältlich ist, wobei das Polynukleotidmolekül eine Nukleotidsequenz umfaßt, die ein Protein codiert, welches zur TGF-β-Superfamilie gehört mit einer konservierten RXXR-Stelle und 7 konservierten Cysteinresten, wobei das codierte Protein:
(i) durch die menschliche neonatale Fibroblastenlinie CCD34Lu in Reaktion auf IGF-1, PDGF BB, TGF-β oder TNF-α in erhöhten Mengen exprimiert werden kann,
(ii) durch Makrophagen in Reaktion auf GM-CSF, M-CSF, IL-2 oder TNF-α in erhöhten Mengen exprimiert werden kann und
(iii) durch menschliche Nabelvenen-Endothelzellen in Reaktion auf ECGF in erhöhten Mengen exprimiert werden kann.

2. Isoliertes Polynukleotidmolekül nach Anspruch 1, wobei das codierte Protein:
(iv) Wachstumsfaktoraktivität auf Vorhaut-Fibroblasten von menschlichen Babys zeigen kann.

3. Isoliertes Polynukleotidmolekül nach einem der Ansprüche 1 oder 2, wobei das Polynukleotidmolekül eine Nukleotidsequenz umfaßt, die der in Figur 1 gezeigten entspricht.

4. Isoliertes Polynukleotidmolekül nach einem der Ansprüche 1 oder 2, wobei die Nukleotidsequenz einer varianten Nukleotidsequenz, ausgewählt unter a1, b1, b2, d2, dd2, f1, h1 und u2, wie in Figur 20A gezeigt, entspricht.

5. Isoliertes Polynukleotidmolekül nach einem der vorangegangenen Ansprüche, wobei die Nukleotidsequenz, die das Protein codiert, keine Sequenz umfaßt, die den nativen Leader oder das Propeptid codiert.

6. Isoliertes Polynukleotidmolekül nach Anspruch 5, wobei die Nukleotidsequenz, die das Protein codiert, eine Sequenz umfaßt, die einen heterologen Leader codiert.

7. Isoliertes Polynukleotidmolekül nach Anspruch 6, wobei der heterologe Leader der Leader des Follikel-stimulierenden Hormons (FSH) ist.

8. Vektor, welcher ein Polynukleotidmolekül nach einem der vorangegangenen Ansprüche umfaßt, welches funktionsfähig mit einem geeigneten Promotor verknüpft ist.

9. Vektor, welcher ein Nukleotidmolekül nach Anspruch 8 umfaßt, wobei das Polynukleotidmolekül funktionsfähig in entgegengesetzter Orientierung mit einem geeigneten Promotor verknüpft ist, so daß die Expression 5' zum 3'-Terminus abläuft, um Antisense-RNA zu erzeugen.

10. Vektor nach Anspruch 9, wobei das Polynukleotidmolekül eine Nukleotidsequenz, die eine Ribozym-Domäne codiert, beinhaltet oder damit verknüpft ist.

11. Protein, welches durch ein Polynukleotidmolekül nach einem der Ansprüche 1 bis 7 codiert wird.

12. Organismus, der mit einem Polynukleotidmolekül nach einem der Ansprüche 1 bis 7 oder einem Vektor nach einem der Ansprüche 8 bis 10 transformiert ist, wobei der Organismus unter eukaryotischen Zellinien, Hefe und Pflanzen ausgewählt ist.

13. Antikörper oder Fragment davon, welcher bzw. welches spezifisch an das Protein nach Anspruch 11 bindet.

14. Protein oder antigener Abschnitt davon, welches bzw. welcher an den Antikörper nach Anspruch 13 bindet.

15. Verfahren zur Herstellung eines Proteins nach Anspruch 11, welches das Transformieren eines geeigneten Wirtsorganismus, ausgewählt unter eukaryotischen Zellinien, Hefe und Pflanzen, mit einem Polynukleotidmolekül nach einem der Ansprüche 1 bis 7 umfaßt, wobei das Polynukleotidmolekül konstitutiv oder induzierbar in dem Wirtsorganismus exprimiert wird.

16. Pharmazeutische Zusammensetzung, welche ein Protein nach Anspruch 11 oder ein Protein oder einen antigenen Abschnitt davon nach Anspruch 14 im Gemisch mit einem pharmazeutisch verträglichen Träger umfaßt.

17. Protein nach Anspruch 11 oder Protein oder antigener Abschnitt davon nach Anspruch 14 zur Verwendung bei der Behandlung einer Erkrankung oder eines Zustands, ausgewählt aus der Gruppe, bestehend aus Wund- und/oder Frakturheilung, ischämischer Verletzung, Krebs, Autoimmunerkrankungen, chronischen Entzündungskrankheiten, Immunsuppression, fibrotischen/fibroproliferativen Störungen, wie rheumatoider Arthritis, Artherosklerose, Lungenfibrose, Sklerodermie, Leberzirrhose und Keloiden.

18. Verwendung eines Proteins nach Anspruch 11 oder eines Proteins oder eines antigenen Abschnitts davon nach Anspruch 14 bei der Herstellung einer Zusammensetzung für die Behandlung einer Erkrankung oder eines Zustands, ausgewählt aus der Gruppe, bestehend aus Wund- und/oder Frakturheilung, ischämischer Verletzung, Krebs, Autoimmunerkrankungen, chronischen Entzündungskrankheiten, Immunsuppression, fibrotischen/fibroproliferativen Störungen, wie rheumatoider Arthritis, Artherosklerose, Lungenfibrose, Sklerodermie, Leberzirrhose und Keloiden.

19. Verfahren zur Herstellung eines Proteins nach Anspruch 15, wobei die eukaryotische Zellinie eine Säugerzellinie ist.

20. In vitro Verfahren zum Diagnostizieren einer Erkrankung oder Störung in einem Subjekt, wobei die Erkrankung oder Störung unter Krebs und entzündlichen und fibrotischen Störungen ausgewählt ist, wobei das Verfahren das Detektieren des Vorliegens eines Proteins nach Anspruch 11 umfaßt.

21. Verfahren nach Anspruch 20, wobei die entzündliche und fibrotische Störung rheumatoide Arthritis, Zirrhose oder Atherosklerose ist.

22. Kit zur Verwendung in dem Verfahren nach Anspruch 20 oder 21, wobei der Kit das Protein nach Anspruch 11 und/oder den Antikörper oder das Fragment davon nach Anspruch 13 umfaßt.

23. Kit zur Verwendung in dem Verfahren nach Anspruch 20 oder 21, wobei der Kit Nukleotidprimer für die Nukleotidsequenz nach einem der Ansprüche 1 bis 7 für Tests auf PCR-Basis umfaßt.

24. Protein nach Anspruch 11, wobei das Protein das reife Protein ist.

## Revendications

1. Polynucléotide isolé pouvant être obtenu à partir du chromosome humain 19p13.1, ladite molécule de polynucléotide comprenant une séquence de nucléotides codant pour une protéine appartenant à la superfamille du Tuf-β ayant un site RXXR conservé et 7 résidus cystéine conservés, où ladite protéine codée est capable :
(i) d'être exprimée par la lignée de fibroblastes néonataux humains CCD34Lu à des taux accrus en réponse au IGF-1, PDGF BB, TGF-β ou TNF-α ;
(ii) d'être exprimée par les macrophages à des taux accrus en réponse au GM-CSF, M-CSF, IL-2 ou TNF-α ; et
(iii) d'être exprimée par des cellules endothéliales de veine ombilicale humaine à des taux accrus en réponse au ECGF.

2. Molécule de polynucléotide isolée suivant la revendication 1, dans laquelle la protéine codée est capable :
(iv) de présenter une activité de facteur de croissance sur les fibroblastes de prépuce de nouveau-nés humains.

3. Molécule de polynucléotide isolée suivant la revendication 1 ou 2, ladite molécule de polynucléotide comprenant une séquence de nucléotides correspondant à celle représentée sur la figure 1.

4. Molécule de polynucléotide isolée suivant la revendication 1 ou 2, dans laquelle la séquence de nucléotides correspond à une séquence de nucléotides variante choisie entre a1, b1, b2, d2, dd2, f1, h1 et u2 représentés sur la figure 20A.

5. Molécule de polynucléotide isolée suivant l'une quelconque des revendications précédentes, dans laquelle la séquence de nucléotides codant pour ladite protéine ne comprend pas la séquence codant pour le leader ou propeptide naturel.

6. Molécule de polynucléotide isolée suivant la revendication 5, dans laquelle ladite séquence de nucléotides codant pour la protéine comprend la séquence codant pour un leader hétérologue.

7. Molécule de polynucléotide isolée suivant la revendication 6, dans laquelle ledit leader hétérologue est le leader de folliculostimuline (FSH).

8. Vecteur comprenant une molécule de polynucléotide suivant l'une quelconque des revendications précédentes liée de manière fonctionnelle à un promoteur convenable.

9. Vecteur comprenant une molécule nucléotidique suivant la revendication 8, dans lequel la molécule de polynucléotide est liée de manière fonctionnelle dans le sens opposé à un promoteur convenable de telle sorte que l'expression s'effectue de l'extrémité 5' à l'extrémité 3' pour produire un ARN antisens.

10. Vecteur suivant la revendication 9, dans lequel la molécule de polynucléotide comprend ou est liée à une séquence de nucléotides codant pour un domaine de ribozyme.

11. Protéine codée par une molécule de polynucléotide suivant l'une quelconque des revendications 1 à 7.

12. Organisme transformé avec une molécule de polynucléotide suivant l'une quelconque des revendications 1 à 7 ou un vecteur suivant l'une quelconque des revendications 8 à 10, ledit organisme étant choisi entre des lignées de cellules eucaryotiques, une levure et des plantes.

13. Anticorps ou un de ses fragments qui se lie spécifiquement à la protéine suivant la revendication 11.

14. Protéine ou sa portion antigénique, qui se lie à l'anticorps suivant la revendication 13.

15. Procédé pour la production d'une protéine suivant la revendication 11, comprenant la transformation d'un organisme hôte convenable choisi entre des lignées de cellules eucaryotiques, une levure et des plantes avec une molécule de polynucléotide suivant l'une quelconque des revendications 1 à 7, dans lequel ladite molécule de polynucléotide est exprimée de manière constitutive ou inductible dans ledit organisme hôte.

16. Composition pharmaceutique comprenant une protéine suivant la revendication 11 ou une protéine ou sa portion antigénique suivant la revendication 14 en mélange avec un support pharmaceutiquement acceptable.

17. Protéine suivant la revendication 11 ou protéine ou sa portion antigénique suivant la revendication 14, destinée à être utilisée dans le traitement d'une maladie ou affection choisie dans le groupe consistant en la guérison de plaies et/ou de fractures, une lésion ischémique, un cancer, des maladies auto-immunes, des maladies inflammatoires chroniques, l'immunosuppression, des troubles fibrotiques/fibroprolifératifs tels que la polyarthrite rhumatoïde, l'athérosclérose, la fibrose pulmonaire, la sclérodermie, la cirrhose hépatique et des chéloïdes.

18. Utilisation d'une protéine suivant la revendication 11 ou d'une protéine ou de sa portion antigénique suivant la revendication 14 dans la production d'une composition destinée au traitement d'une maladie ou affection choisie dans le groupe consistant en la guérison de plaies et/ou de fractures, une lésion ischémique, un cancer, des maladies auto-immunes, des maladies inflammatoires chroniques, une immunosuppression, des troubles fibrotiques/fibroprolifératifs tels que la polyarthrite rhumatoïde, l'athérosclérose, la fibrose pulmonaire, la sclérodermie, la cirrhose hépatique et des chéloïdes.

19. Procédé pour la production d'une protéine suivant la revendication 15, dans lequel ladite lignée de cellules eucaryotiques est une lignée de cellules de mammifère.

20. Méthode in vitro pour diagnostiquer une maladie ou un trouble chez un sujet, ladite maladie ou ledit trouble étant choisi entre le cancer et des troubles inflammatoires et fibrotiques, ladite méthode comprenant la détection de la présence d'une protéine suivant la revendication 11.

21. Méthode suivant la revendication 20, dans laquelle le trouble inflammatoire et fibrotique est la polyarthrite rhumatoïde, la cirrhose ou l'athérosclérose.

22. Kit destiné à être utilisé dans la méthode suivant la revendication 20 ou 21, ledit kit comprenant la protéine suivant la revendication 11 et/ou l'anticorps ou son fragment suivant la revendication 13.

23. Kit destiné à être utilisé dans la méthode suivant la revendication 20 ou 21, ledit kit comprenant des amorces nucléotidiques pour une séquence de nucléotides suivant l'une quelconque des revendications 1 à 7 pour des analyses basées sur la PCR.

24. Protéine selon la revendication 11, ladite protéine étant la protéine mature.
